# EUROPEAN PATENT SPECIFICATION

(11) **EP 1 210 460 B1**
(45) Date of publication and mention of the grant of the patent: **23.05.2007**
(21) Application number: 00957571.3
(22) Date of filing: 18.08.2000
(51) Int. Cl.: C12N 15/10, C12N 15/52, C12Q 1/68

(54) **GENE CLONING**
GENKLONIERUNG
CLONAGE DE GENES

(30) Priority: 19.08.1999 US 149788 P; 19.09.1999 US 149822 P
(43) Date of publication of application: 05.06.2002
(73) Proprietor: Omniscience Pharmaceuticals, Tempe, AZ 85282-6764 (US)
(72) Inventor: FOMENKOV, Alexey, Tempe, AZ 85213 (US); HUANG, Yiping, Tempe, AZ 85282 (US); CHAPARIAN, Michael, G., Chandler, AZ 85226 (US); ZHENG, Shu-Xian, Peoria, AZ 85382 (US)
(74) Representative: Pidgeon, Robert John
(86) International application number: PCT/US2000/022743
(87) International publication number: WO 2001/012861

(56) References cited:
- WO-A-92/07078
- WO-A-94/24277
- WO-A-95/04150
- WO-A-96/34112
- WO-A-96/40968
- WO-A-97/33988
- WO-A-98/17811
- WO-A-99/10539
- US-A- 5 521 077
- US-A- 5 728 561

### SEQUENCE LISTING

<110> OMNISCIENCE PHARMACEUTICALS
   Fomenkov, Alexey
   Huang, Yiping
   Chaparian, Michael
   Zheng, Shu-Xian
<120> GENE CLONING
<130> 1002.00012
<140> 00957571.3
   <141> 2000-08-18
<150> PCT/US00/22743
   <151> 2000-08-18
<150> 60/149,788
   <151> 1999-08-19
<150> 60/149,822
   <151> 1999-08-19
<160> 141
<170> Patent In version 3.0
<210> 1
   <211> 29
   <212> DNA
   <213> artificial sequence
<220>
   <221> misc_feature
   <222> (1)..(29)
   <223> primer
<400> 1
   gggtccggca gaccgttcgc gggccggac
   29
<210> 2
   <211> 30
   <212> DNA
   <213> artificial sequence
<220>
   <221> misc_feature
   <222> (1) .. (30)
   <223> primer
<400> 2
   gagcggaccg caccgcgatc ggaacaacct 30
<210> 3
   <211> 28
   <212> DNA
   <213> artificial sequence
<220>
   <221> misc_feature
   <222> (1)..(28)
   <223> primer
<400> 3
   tctccggggc agcgcggtcg cggaacgt 28
<210> 4
   <211> 8
   <212> DNA
   <213> artificial sequence
<220>
   <221> misc_feature
   <222> (1)..(8)
   <223> Octamer OC-OCT-003
<400> 4
   ctcgccga 8
<210> 5
   <211> 8
   <212> DNA
   <213> artificial sequence
<220>
   <221> misc_feature
   <222> (1) .. (8)
   <223> octamer OS-OCT-001
<400> 5
   gtcggcga 8
<210> 6
   <211> 8
   <212> DNA
   <213> artificial sequence
<220>
   <221> misc_feature
   <222> (1) .. (8)
   <223> octamer OS-OCT-002
<400> 6
   ccagatcg
   8
<210> 7
   <211> 8
   <212> DNA
   <213> artificial sequence
<220>
   <221> misc_feature
   <222> (1)..(8)
   <223> octamer OC-OCT004
<400> 7
   cgacatcg
   8
<210> 8
   <211> 8
   <212> DNA
   <213> artificial sequence
<220>
   <221> misc_feature
   <222> (1)..(8)
   <223> octamer OS-OCT-005
<400> 8
   gccgatca
   8
<210> 9
   <211> 8
   <212> DNA
   <213> artificial sequence
<220>
   <221> misc_feature
   <222> (1) .. (8)
   <223> octamer OS-OCT-006
<400> 9
   gccaccga
   8
<210> 10
   <211> 8
   <212> DNA
   <213> artificial seqeunce
<220>
   <221> misc_feature
   <222> (1) .. (8)
   <223> octamer OS-OCT-007
<400> 10
   gatgccga
   8
<210> 11
   <211> 8
   <212> DNA
   <213> artificial sequence
<220>
   <221> misc_feature
   <222> (1)..(8)
   <223> octmer OS-OCT-008
<400> 11
   cggcgaag
   8
<210> 12
   <211> 8
   <212> DNA
   <213> artificial sequence
<220>
   <221> misc_feature
   <222> (1)..(8)
   <223> octamer OS-OCT-009
<400> 12
   cggcgaac
   8
<210> 13
   <211> 8
   <212> DNA
   <213> artificial sequence
<220>
   <221> misc_feature
   <222> (1) .. (8)
   <223> octamer OS-OCT-010
<400> 13
   ggcgatca
   8
<210> 14
   <211> 8
   <212> DNA
   <213> artificial sequence
<220>
   <221> misc_feature
   <222> (1)..(8)
   <223> octamer OS-OCT-011
<400> 14
   gccgagga
   8
<210> 15
   <211> 8
   <212> DNA
   <213> artificial sequence
<220>
   <221> misc_feature
   <222> (1)..(8)
   <223> octamer OS-OCT-012
<400> 15
   cgccgaca
   8
<210> 16
   <211> 8
   <212> DNA
   <213> artificial sequence
<220>
   <221> misc_feature
   <222> (1) .. (8)
   <223> octamer OS-OCT-013
<400> 16
   atcgccga
   8
<210> 17
   <211> 8
   <212> DNA
   <2.13> artificial sequence
<220>
   <221> misc_feature
   <222> (1) .. (8)
   <223> octamer OS-OCT-014
<400> 17
   ggcgaacc
   8
<210> 18
   <211> 8
   <212> DNA
   <213> artificial sequence
<220>
   <221> misc_feature
   <222> (1)..(8)
   <223> octamer OS-OCT-015
<400> 18
   gccgacca
   8
<210> 19
   <211> 8
   <212> DNA
   <213> artificial sequence
<220>
   <221> misc_feature
   <222> (1)..(8)
   <223> octamer OS-OCT-016
<400> 19
   gccaagga
   8
<210> 20
   <211> 8
   <212> DNA
   <213> artificial sequence
<220>
   <221> misc_feature
   <222> (1)..(8)
   <223> octamer OS-OCT-017
<400> 20
   cggcaacg
   8
<210> 21
   <211> 8
   <212> DNA
   <213> artificial sequence
<220>
   <221> misc_feature
   <222> (1) .. (8)
   <223> octamer OS-OCT-018
<400> 21
   ggctggac
   8
<210> 22
   <211> 8
   <212> DNA
   <213> artificial sequence
<220>
   <221> misc_feature
   <222> (1)..(8)
   <223> octamer OS-OCT-019
<400> 22
   gcagcacc
   8
<210> 23
   <211> 8
   <212> DNA
   <213> artificial sequence
<220>
   <221> artificial sequence
   <222> (1) .. (8)
   <223> octamer OS-OCT-020
<400> 23
   ccagccag
   8
<210> 24
   <211> 8
   <212> DNA
   <213> artificial sequence
<220>
   <221> misc_feature
   <222> (1)..(8)
   <223> octamer OS-OCT-21
<400> 24
   cgccgccg
   8
<210> 25
   <211> 8
   <212> DNA
   <213> artificial sequence
<220>
   <221> misc_feature
   <222> (1)..(8)
   <223> octamer OS-OCT-22
<400> 25
   cggcgacc
   8
<210> 26
   <211> 8
   <212> DNA
   <213> artificial sequence
<220>
   <221> misc_feature
   <222> (1) .. (8)
   <223> octamer OS-OCT-23
<400> 26
   ccgccgcc
   8
<210> 27
   <211> 8
   <212> DNA
   <213> artificial sequence
<220>
   <221> misc_feature
   <222> (1) .. (8)
   <223> octamer OS-OCT-24
<400> 27
   cgcggccg
   8
<210> 28
   <211> 8
   <212> DNA
   <213> artificial sequence
<220>
   <221> misc_feature
   <222> (1)..(8)
   <223> octamer OS-OCT-25
<400> 28
   gtcggcga
   8
<210> 29
   <211> 10
   <212> DNA
   <213> artificial sequence
<220>
   <221> misc_feature
   <222> (1)..(10)
   <223> decamer OS-DEC-001
<400> 29
   cagctcggcg
   10
<210> 30
   <211> 10
   <212> DNA
   <213> artificial sequence
<220>
   <221> misc_feature
   <222> (1)..(10)
   <223> decamer OS-DEC-002
<400> 30
   gccggtgagc
   10
<210> 31
   <211> 10
   <212> DNA
   <213> artificial sequence
<220>
   <221> misc_feature
   <222> (1)..(10)
   <223> decamer OS-DEC-003
<400> 31
   ccgggtcgag
   10
<210> 32
   <211> 10
   <212> DNA
   <213> artificial sequence
<220>
   <221> misc_feature
   <222> (1)..(10)
   <223> decamer OS-DEC-004
<400> 32
   ggcgccgccc
   10
<210> 33
   <211> 10
   <212> DNA
   <213> artificial sequence
<220>
   <221> misc_feature
   <222> (1) .. (10)
   <223> decamer OS-DEC-005
<400> 33
   ggcgccgccc
   10
<210> 34
   <211> 10
   <212> DNA
   <213> artificial sequence
<220>
   <221> misc feature
   <222> (1)..(10)
   <223> decamer OS-DEC-006
<400> 34
   cgaggtcgag
   10
<210> 35
   <211> 10
   <212> DNA
   <213> artificial sequence
<220>
   <221> misc_feature
   <222> (1)..(10)
   <223> decamer OS-DEC-007
<400> 35
   cgagcaggcc
   10
<210> 36
   <211> 10
   <212> DNA
   <213> artificial sequence
<220>
   <221> misc_feature
   <222> (1)..(10)
   <223> decamer OS-DEC-008
<400> 36
   cgacgcgggc
   10
<210> 37
   <211> 10
   <212> DNA
   <213> artificial sequence
<220>
   <221> misc_feature
   <222> (1) .. (10)
   <223> decamer OC-DEC-009
<400> 37
   cctggccgcg
   10
<210> 38
   <211> 10
   <212> DNA
   <213> artificial sequence
<220>
   <221> misc_feature
   <222> (1)..(10)
   <223> decamer OS-DEC-010
<400> 38
   cctgcgcggc
   10
<210> 39
   <211> 10
   <212> DNA
   <213> artificial sequence
<220>
   <221> misc_feature
   <222> (1) .. (10)
   <223> decamer OS-DEC-011
<400> 39
   acggccgcgg
   10
<210> 40
   <211> 10
   <212> DNA
   <213> artificial sequence
<220>
   <221> misc_feature
   <222> (1) .. (10)
   <223> decamer OS-DEC-012
<400> 40
   cgaggacgtc
   10
<210> 41
   <211> 20
   <212> PRT
   <213> artificial sequence
<220>
   <221> UNSURE
   <222> (1)..(20)
   <223> primer
<400> 41
   Cys Gly Ala Thr Gly Cys Thr Ser Thr Ala Tyr Gly Ala Arg Thr Gly
   1               5                   10                  15
   Gly Cys Thr Ala
               20
<210> 42
   <211> 20
   <212> PRT
   <213> artificial sequence
<220>
   <221> UNSURE
   <222> (1) .. (20)
   <223> primer
<400> 42
   Thr Gly Gly Cys Gly Tyr Gly Thr Tyr Thr Gly Val Ala Cys Cys Ala
   1               5                   10                  15
   Thr Gly Thr Ala
               20
<210> 43
   <2.11> 20
   <.212> PRT
   <213> artificial sequence
<220>
   <221> UNSURE
   <222> (1) .. (20)
   <223> primer
<400> 43
<210> 44
   <211> 20
   <212> PRT
   <213> artificial sequence
<220>
   <221> UNSURE
   <222> (1)..(20)
   <223> primer
<400> 44
   Cys Ala Thr Cys Asx Gly Gly Ser Gly Thr Ser Gly Thr Thr Ala Cys
   1               5                   10                  15
   Gly Gly Thr Ala
               20
<210> 45
   <211> 21
   <212> PRT
   <213> artificial sequence
<220>
   <221> unsure
   <222> (1)..(20)
   <223> primer
<400> 45
   Thr Gly Cys Thr Ser Gly Thr Ser Gly Gly Ser Gly Ala Arg Gly Ala
   1               5                   10                  15
   Gly Cys Thr Gly Ala
               20
<210> 46
   <211> 22
   <212> PRT
   <213> artificial sequence
<220>
   <221> UNSURE
   <222> (1)..(22)
   <223> primer
<400> 46
   Thr Cys Ser Ala Cys Tyr Thr Thr Gly Cys Cys Arg Thr Thr Gly Ala
   1               5                   10                  15
   Cys Arg Thr Thr Gly Ala
               20
<210> 47
   <211> 22
   <212> PRT
   <213> artificial sequence
<220>
   <221> UNSURE
   <222> (1)..(22)
   <223> primer
<400> 47
   Thr Thr Cys Ala Cys Cys Gly Ala Arg Arg Cys Ser Gly Cys Gly Thr
   1               5                   10                  15
   Thr Cys Gly Thr Cys Ala
               20
<210> 48
   <211> 23
   <212> PRT
   <213> artificial sequence
<220>
   <221> UNSURE
   <222> (1) .. (23)
   <223> primer
<400> 48
   Cys Gly Cys Cys Gly Gly Ser Ala Cys Cys Ala Thr Ser Ala Tyr Cys
   1               5                   10                  15
   Cys Gly Gly Ala Thr Ser Ala
               20
<210> 49
   <211> 19
   <212> PRT
   <213> artificial sequence
<220>
   <221> UNSURE
   <222> (1)..(19)
   <223> primer
<400> 49
   Gly Ala Thr Cys Gly Cys Gly Thr Gly Cys Gly Cys Ala Ala Gly Ala
   1               5                   10                  15
   Ala Ala Thr
<210> 50
   <211> 22
   <212> PRT
   <213> artificial sequence
<220>
   <221> UNSURE
   <222> (1) .. (22)
   <223> primer
<400> 50
   Cys Cys Ala Cys Ser Thr Thr Thr Gly Gly Tyr His Thr Arg Gly Gly
   1               5                   10                  15
   Arg Gly Ala Thr Cys Gly
               20
<210> 51
   <211> 24
   <212> PRT
   <213> artificial sequence
<220>
   <221> UNSURE
   <222> (1) .. (24)
   <223> primer
<400> 51
   Ala Tyr Arg Cys Gly Thr Thr Cys Ala Ala Gly Tyr Gly Cys Met Gly
   1               5                   10                  15
   Cys Met Met Met Ala Cys Ala Gly
               20
<210> 52
   <211> 20
   <212> PRT
   <213> artificial sequence
<220>
   <221> UNSURE
   <222> (1)..(20)
   <223> primer
<400> 52
   Gly Ala Thr Ala Ala Ala Thr Tyr Thr Gly Tyr Ala Cys Thr Gly Ala
   1               5                   10                  15
   Arg Cys Cys Lys
               20
<210> 53
   <211> 23
   <212> PRT
   <213> artificial sequence
<220>
   <221> UNSURE
   <222> (1)..(23)
   <223> primer
<400> 53
   Thr Thr Cys Lys Ser Cys Gly Ala Gly Gly Ala Cys Cys Ala Cys Cys
   1               5                   10                  15
   Cys Gly Met Trp Gly Ala Thr
               20
<210> 54
   <211> 21
   <212> PRT
   <213> artificial sequence
<220>
   <221> UNSURE
   <222> (1)..(21)
   <223> primer
<400> 54
   Gly Gly Ala Ala Gly Thr Ala Gly Thr Cys Gly Thr Lys Ser Gly Thr
   1               5                   10                  15
   Gly Ala Lys Gly Thr
               20
<210> 55
   <211> 20
   <212> PRT
   <213> artificial sequence
<220>
   <221> UNSURE
   <222> (1)..(20)
   <223> primer
<400> 55
   Gly Ala Thr Gly Cys Ala Cys Gly Ala Gly Gly Thr Asx Ala Ala Cys
   1               5                   10                  15
   Val Thr Cys Thr
               20
<210> 56
   <211> 21
   <212> PRT
   <213> artificial sequence
<220>
   <221> UNSURE
   <222> (1)..(21)
   <223> primer
<400> 56
   Thr Cys Thr Gly Gly Trp Ala Ser Ala Gly Ser Ala Cys Gly Gly Thr
   1               5                   10                  15
   Gly Ala Thr Cys Ala
               20
<210> 57
   <211> 22
   <212> PRT
   <213> artificial sequence
<220>
   <221> UNSURE
   <222> (1) .. (22)
   <223> primer
<400> 57
   Ala Thr Gly Cys Ser Arg Cys Gly Tyr Ala Ala Arg Cys Gly Gly Cys
   1               5                   10                  15
   Ala Arg Thr Thr Gly Thr
               20
<210> 58
   <211> 22
   <212> PRT
   <213> artificial sequence
<220>
   <221> UNSURE
   <222> (1)..(22)
   <223> primer
<400> 58
   Cys Gly Ala Thr Arg Gly Cys Tyr Thr Cys Tyr Thr Thr Gly Cys Cys
   1               5                   10                  15
   Tyr Gly Thr Cys Ala Thr
               20
<210> 59
   <211> 22
   <212> PRT
   <213> artificial sequence
<220>
   <221> UNSURE
   <222> (1)..(22)
   <223> primer
<400> 59
   Cys Gly Thr Thr Gly Gly Cys Tyr Cys Thr Thr Cys Ala Thr Gly Gly
   1 5 10 15
   Ser Gly Ala Thr Gly Ala
   20
<210> 60
   <211> 23
   <212> PRT
   <213> artificial sequence
<220>
   <221> UNSURE
   <222> (1)..(23)
   <223> primer
<400> 60
   Cys Cys Thr Thr Thr Gly Gly Cys Thr Ala Thr Tyr Thr Cys Arg Gly
   1 5 10 15
   Cys Tyr Thr Cys Cys Ala Thr
   20
<210> 61
   <211> 20
   <212> PRT
   <213> artificial sequence
<220>
   <221> UNSURE
   <222> (1)..(20)
   <223> primer
<400> 61
   Gly Ala Gly Thr Thr Cys Gly Ala Cys Gly Cys Ser Gly Val Ser Thr
   1 5 10 15
   Thr Cys Thr Thr
   20
<210> 62
   <211> 21
   <212> PRT
   <213> artificial sequence
<220>
   <221> UNSURE
   <222> (1)..(21)
   <223> primer
<400> 62
   Gly Gly Thr Gly Thr Gly Asn Cys Cys Gly Ala Thr Gly Thr Thr Gly
   1 5 10 15
   Gly Ala Cys Thr Thr
   20
<210> 63
   <211> 20
   <212> PRT
   <213> artificial sequence
<220>
   <221> UNSURE
   <222> (1) .. (20)
   <223> primer
<400> 63
   Gly Cys Ala Arg Cys Gly Val Cys Thr Cys Cys Thr Gly Cys Thr Ser
   1 5 10 15
   Gly Ala Ala Ala
   20
<210> 64
   <211> 20
   <212> PRT
   <213> artificial sequence
<220>
   <221> UNSURE
   <222> (1)..(20)
   <223> primer
<400> 64
   Thr Thr Gly Cys Thr Ser Gly Cys Arg Cys Cys Gly Thr Cys Cys Thr
   1               5                   10                  15
   Gly Gly Thr Thr
               20
<210> 65
   <211> 20
   <212> PRT
   <213> artificial sequence
<220>
   <221> UNSURE
   <222> (1) .. (20)
   <223> primer
<400> 65
   Ala Gly Cys Gly Thr Ser Thr Thr Cys Gly Cys Ser Gly Thr Gly Gly
   1               5                   10                  15
   Ala Gly Thr Ala
               20
<210> 66
   <211> 20
   <212> PRT
   <213> artificial sequence
<220>
   <221> UNSURE
   <222> (1) .. (20)
   <223> primer
<400> 66
   Gly Thr Gly Cys Thr Thr Cys Thr Cys Ser Ala Gly Met Ala Gly Cys
   1               5                   10                  15
   Thr Thr Gly Ala
               20
<210> 67
   <211> 20
   <212> PRT
   <213> artificial sequence
<220>
   <221> UNSURE
   <222> (1) .. (20)
   <223> primer
<400> 67
   Thr Ser Ala Ala Gly Gly Ala Gly Ala Cys Cys Gly Ala Arg Gly Ala
   1               5                   10                  15
   Ser Gly Ala Ala
               20
<210> 68
   <211> 19
   <212> PRT
   <213> artificial sequence
<220>
   <221> UNSURE
   <222> (1)..(19)
   <223> primer
<400> 68
   Cys Gly Ser Ala Cys Cys Ala Cys Ser Ala Gly Cys Ala Gly Gly Thr
   1               5                   10                  15
   Thr Cys Ala
<210> 69
   <211> 20
   <2.12> PRT
   <213> artificial sequence
<220>
   <221> UNSURE
   <222> (1)..(20)
   <223> primer
<400> 69
   Cys Thr Thr Cys Thr Ala Tyr Gly Ser Val Cys Thr Gly Gly Ala Gly
   1               5                   10                  15
   Gly Cys Cys Ala
               20
<210> 70
   <211> 20
   <212> PRT
   <213> artificial sequence
<220>
   <221> UNSURE
   <222> (1) .. (20)
   <223> primer
<400> 70
   Gly Ala Gly Gly Arg Gly Ala Thr Cys Thr Gly Ser Ser Cys Cys Asp
   1               5                   10                  15
   Thr Gly Cys Thr
               20
<210> 71
   <211> 20
   <212> PRT
   <213> artificial sequence
<220>
   <221> UNSURE
   <222> (1)..(20)
   <223> primer
<400> 71
   Gly Thr Gly Gly Gly Met Gly Ala Cys Gly Gly Tyr Thr Cys Ser Ala
   1               5                   10                  15
   Ala Arg Thr Thr
               20
<210> 72
   <211> 20
   <212> PRT
   <213> artificial sequence
<220>
   <221> UNSURE
   <222> (1)..(20)
   <223> primer
<400> 72
<2.10> 73
   <211> 20
   <212> PRT
   <213> artificial sequence
<220>
   <221> UNSURE
   <222> (1) .. (20)
   <223> primer
<400> 73
<210> 74
   <211> 19
   <212> PRT
   <213> artificial sequence
<220>
   <221> UNSURE
   <222> (1)..(19)
   <223> primer
<400> 74
   Gly Cys Cys Ala Lys Asx Cys Cys Gly Thr Cys Gly Ser Tyr Cys Ala
   1 5 10 15
   Gly Gly Thr
<210> 75
   <211> 19
   <212> PRT
   <213> artificial sequence
<220>
   <221> UNSURE
   <222> (1) .. (19)
   <223> primer
<400> 75
   Cys Ser Gly Tyr Ser Thr Thr Cys Gly Thr Gly Cys Gly Gly Ala Cys
   1 5 10 15
   Cys Ala Ala
<210> 76
   <211> 19
   <212> PRT
   <213> artificial sequence
<220>
   <221> UNSURE
   <22.2> (1)..(19)
   <223> primer
<400> 76
   Cys Gly Gly Thr Cys Ser Lys Ser Gly Ala Cys Gly Thr Arg Cys Thr
   1               5                   10                  15
   Cys Cys Ala
<210> 77
   <211> 20
   <212> PRT
   <213> artificial sequence
<220>
   <221> UNSURE
   <222> (1)..(20)
   <223> primer
<400> 77
   Ala Thr Cys Gly Thr Ser Cys Cys Tyr Gly Gly Arg Thr Ala Ser Ala
   1               5                   10                  15
   Thr Gly Thr Thr
               20
<210> 78
   <211> 19
   <212> PRT
   <213> artficial sequence
<220>
   <221> UNSURE
   <222> (1)..(19)
   <223> primer
<400> 78
   Gly Ala Thr Gly Gly Ala Arg Cys Gly Ser Cys Cys Ser Ala Gly Ser
   1               5                   10                  15
   Ala Cys Ala
<210> 79
   <211> 23
   <212> PRT
   <213> artificial sequence
<220>
   <221> UNSURE
   <222> (1) .. (23)
   <223> primer
<400> 79
   Cys Thr Thr Ser Thr Cys Cys Cys Thr Ser Ala Ala Tyr Cys Ala Ser
   1 5 10 15
   Thr Ala Tyr Ala Ala Gly Ala
   20
<210> 80
   <211> 20
   <212> PRT
   <213> artificial sequence
<220>
   <221> UNSURE
   <222> (1)..(20)
   <223> primer
<400> 80
   Gly Cys Gly Thr Thr Cys Cys Arg Gly Gly Tyr Thr Cys Arg Ala Ala
   1 5 10 15
   Gly Gly Ala Ala
   20
<210> 81
   <211> 21
   <212> PRT
   <213> artificial sequence
<220>
   <221> UNSURE
   <222> (1) .. (21)
   <223> primer
<400> 81
   Gly Cys Met Thr Ser Cys Cys Ser Ser Thr Ser Ala Thr Cys Gly Ala
   1 5 10 15
   Gly Gly Ala Cys Thr
   20
<210> 82
   <211> 21
   <212> PRT
   <213> artificial sequence
<220>
   <221> UNSURE
   <222> (1)..(21)
   <223> primer
<400> 82
   Gly Thr Cys Gly Ala Ala Cys Cys Gly Gly Gly Ser Met Ser Gly Gly
   1               5                   10                  15
   Gly Thr Cys Cys Ala
               20
<210> 83
   <211> 19
   <212> PRT
   <213> artificial sequence
<220>
   <221> UNSURE
   <222> (1)..(19)
   <223> primer
<400> 83
   Cys Gly Gly Cys Gly Cys Arg Tyr Ser Gly Gly Arg Gly Thr Ser Thr
   1               5                   10                  15
   Thr Cys Ala
<210> 84
   <211> 20
   <212> PRT
   <213> artificial sequence
<220>
   <221> UNSURE
   <222> (1)..(20)
   <223> primer
<400> 84
   Gly Thr Trp Cys Ala Gly Cys Gly Gly Gly Thr Thr Gly Lys Cys Gly
   1               5                   10                  15
   Thr Thr Cys Ala
               20
<210> 85
   <211> 21
   <212> PRT
   <213> artificial sequence
<220>
   <221> UNSURE
   <222> (1)..(21)
   <223> primer
<400> 85
   Ala Thr Gly Ala Thr Gly Thr Gly Gly Gly Thr Tyr Thr Gly Ser Thr
   1 5 10 15
   Cys Ser Ala Gly Ala
   20
<210> 86
   <211> 21
   <212> PRT
   <213> artificial sequence
<220>
   <221> UNSURE
   <222> (1) .. (21)
   <223> primer
<400> 86
   Thr Thr Thr Tyr Thr Cys Arg Thr Cys Ser Cys Cys Thr Gly Thr Arg
   1 5 10 15
   Thr Thr Cys Ala Thr
   20
<210> 87
   <211> 21
   <212> PRT
   <213> artificial sequence
<220>
   <22.1> UNSURE
   <222> (1)..(21)
   <223> primer
<400> 87
   Thr Cys Cys Tyr Gly Gly Tyr Cys Cys Lys Gly Thr Ser Gly Thr Met
   1 5 10 15
   Ala Thr Gly Ala Thr
   20
<210> 88
   <211> 21
   <212> PRT
   <213> artificial sequence
<220>
   <221> UNSURE
   <222> (1)..(21)
   <223> primer
<400> 88
   Ala Cys Cys Tyr Thr Cys Ser Ala Ala Ser Gly Cys Arg Thr Thr Tyr
   1 5 10 15
   Ala Ala Cys Ala Thr
   20
<210> 89
   <211> 290
   <212> DNA
   <213> artificial sequence
<220>
   <221> misc_feature.
   <222> (1) .. (290)
   <223> probe
<400> 89
   ccgacactcg cggacgtaca gacatgaacc tggcgcttgg gttagaagaa gataccggtc 60
   tagagcactt gaagtaggag gaggccggaa ggggatgggg aaaacgctct cacaggtgga 120
   caagggaacg aggcagggtc ttatcttaag gctgatctcc gagaagcatt ggggaccagc 180
   aggatctggt cgcggccctg tcggaggctg gggttgaggt ggcccaggcg accgtgagtc 240
   gggaccttgc gagctcgggg tcctaaaggt cggtaaccgc tatctccggc 290
<210> 90
   <211> 267
   <212> DNA
   <213> artificial sequence
<220>
   <221> misc_feature
   <222> (1) .. (267)
   <223> probe
<400> 90
   ccgacgcttg ctgacgtaca ggcggaatgc agaacttcca cgggggcatt aaaacgttca
   60
   cgaaaacggg cgatagtttg cggtgtcagg ccgatttccg gcaccatcac cagcgcctgt
   120
   ttgccctgag cgagcacgtt ttccagtacg ctgagataaa cctccgtttt acgtaaccac
   180
   gcccgatgat cacgaattct ggatccgata cgtaacgcgt ctgcagcatg cgtggtaccg
   240
   agctttccct atagtgagtc gtataga
   267
<210> 91
   <211> 274
   <212> DNA
   <213> artificial sequence
<220>
   <221> misc_feature
   <222> (1)..(274)
   <223> probe
<400> 91
   catctggggt ggttacggta caggcaactt cacgccatct gccgagttca acatctcttg
   60
   ccgacccaga agccgcacgc gtagtgttca cctccggcgt tccattagtg atgatgggcc
   120
   tcgatctcac aaccagaccg tttgcacccg gacgtgattg ctcggatgga aaggcaggcg
   180
   gcccgccgga gagctgttca gcgacatcat gaacttcact ctcaaaacgc agtcgaaaac
   240
   tacggccttg ctggcggccg gtgcacgacg ccac
   274
<210> 92
   <211> 293
   <212> DNA
   <213> artificial sequence
<220>
   <221> misc_feature
   <222> (1) .. (293)
   <223> probe
<400> 92
   ccgacgctcg cggacgtaca tgacaaacct ttatttcaag attaaagaag ataagcgcaa 60
   ggctgcgaga ggtgaataat gcctccatca cttacgcaaa agccgcttgc tgctgctcat 120
   tggtggcgcg acgcaattgc tcatagcact cacgtgttaa tcactcggcc caatggtaac 180
   cgatggtccc tggaagatgt ccagccctac cataccatca ccaaagatat tgttggtgta 240
   tggcactgta tgctcaccgg acacaccgga aaagaccatc attgctccgg taa 293
<210> 93
   <211> 95
   <212> DNA
   <213> artificial sequence
<220>
   <221> misc_feature
   <222> (1) .. (95)
   <223> probe
<400> 93
   gaggcgatat cattttctac aggaatacgc accaaagact caatcagatt gcgtccaaca 60
   agaccggcat tgcatcgggg gtggttacgg tattc 95
<210> 94
   <211> 105
   <212> DNA
   <213> artificial sequence
<220>
   <221> misc_feature
   <222> (1)..(105)
   <223> probe
<400> 94
   catcgggggt ggttacggta taaactgcgg cttcttcttt ttcttctttc ttcttgttac 60
   acgctgtaaa caacagaaga ctgcttagcg caatacttgc gacaa 105
<210> 95
   <211> 270
   <212> DNA
   <213> artificial sequence
<220>
   <221> misc_feature
   <222> (1)..(270)
   <223> probe
<400> 95
   ccgacgctcg cggacgtaca tcttcaagcg tccatcgctc ggcatggtga tgtggatctg 60
   gatcagcgtg atgaacccgc atacgcaagg gtggggcttc gcgcgcgaag cgttcgccgc 120
   catcatcgcg gtgacgacgg tcgccgccat ggccacgaac gcgtaccgga ataccgtaac 180
   cacccccgat gatcacgaat tctggatccg atacgtaacg cgtctgcagc atgcgtggta 240
   cgagctttcc tatagtgagt cgtatagagg 270
<210> 96
   <211> 126
   <212> DNA
   <213> artificial sequence
<220>
   <221> misc_feature
   <222> (1)..(126)
   <223> probe
<400> 96
   gatcgcgtgc gcaagaaatc tgcgccgcct ggcagggtcg agttgtcggc tggtactgca 60
   cagatctgac ccctgaaggc tatgccgtcg agtccgagtc tcaccccggc tcagtacaga 120
   tttatc 126
<210> 97
   <211> 127
   <212> DNA
   <213> artificial sequence
<220>
   <221> misc_feature
   <222> (1)..(127)
   <223> probe
<400> 97
   gataaatatg cactgagccg gggtgagact cggactcgac ggcatagcct tcaggggtca 60
   gatttgtgca gtaccagccg acaactcgac cctgccaggc ggcgccagat ttcttgcgca 120
   cgcgatc 127
<210> 98
   <211> 127
   <212> DNA
   <213> artificial sequence
<220>
   <221> misc_feature
   <222> (1)..(127)
   <223> probe
<400> 98
   gataaatctg tactgagcct ggatgcgact cggactcgac ggcatagcct tcaggggtca 60
   gttttgtgca gtaccagccg acaactcgac cctgccaggc ggcgccagat ttcttgcgca 120
   cgcgatc 127
<210> 99
   <211> 275
   <212> DNA
   <213> artificial sequence
<220>
   <221> misc_feature
   <222> (1) .. (275)
   <223> probe
<400> 99
   cctttggcta tttcggcttc catctcgacg acagtgaagt tctccctgta gaagaagtcg
   60
   aggtacccct cgttctgaag aaatgtccct ttgaccgtgg accgcctttt ggttatcgag
   120
   cgcggcgcca taatccgagg gtatgggggc gaggtcggca taggctggaa cgcatttcgg
   180
   aaccaggtag gtgggttccc gggaggtggc ctcggtcata ggacaacgtc cgaggatcat
   240
   tcacgtcgcc caatgggcgg cccggttggg gccgt
   275
<210> 100
   <211> 286
   <212> DNA
   <213> artificial sequence
<220>
   <221> misc_feature
   <222> (1)..(286)
   <223> probe
<400> 100
   cctttggcta tttcggcttc catcgtggcc tccttgcctg tcatccactt cttcaacaga
   60
   gatatttgag aaatcagaaa tttctgtctt taaaggagat gtctggctgc gggaaccgat
   120
   catctgtagc tgtgttctta taatattctg aatttttgca cgcttgtttc ttctgctttt
   180
   ttttctaaag ataccagaat agcaaccaaa ggcagcaagc agtacaacaa ctgccgtttg
   240
   gcgccgcata tctgaattcg tcgacaagct tcttgagcct aggcta
   286
<210> 101
   <211> 272
   <212> DNA
   <213> artificial sequence
<220>
   <221> misc_feature
   <222> (1)..(272)
   <223> probe
<400> 101
   cctttggcta tttcggcttc catgccgtca tgacaccctc ctggtgttcg tacaattttt 60
   cttttatcac ctttgcgccc tgttcttctt ctacaccgtc aacggactta ctaccatcgg 120
   taaatggccg cggcgtatca tattcgccct cttatttctc aaccctgcca tcctttatct 180
   caggtaacta tatcaccagg tgacgtctat ttcatcgcca tgaagggccc acgatctgaa 240
   ttcgtcgaca aggcttctcg agcctagggc ta 272
<210> 102
   <211> 101
   <212> DNA
   <213> artificial sequence
<220>
   <221> misc_feature
   <222> (1)..(101)
   <223> probe
<400> 102
   cgatggcctc tttgcctgtc atttttcgat cactaccacc gggcgtgcca gtcgtattgc 60
   cagcgcctgt gccgtctcgc cttgtgtcta atcaataaaa c 101
<210> 103
   <211> 262
   <212> DNA
   <213> artificial sequence
<220>
   <221> misc_feature
   <222> (1) .. (262)
   <223> probe
<400> 103
   cgttggccct tcatggcgat gatgtcagca ccacgcctgt cgcggcgctc aaaagatagc 60
   tgtggccgag catgacggga aacatgctgc gatcctgtgc gacacggcgg atcagcgatt 120
   cctgcgaacc gataccgcag atctggacgc caagattggt gacggccgta tggaggcgta 180
   aagcgcgaat tgttcgacac cgagatgacg ggcaaggagg ccatcatcgc catgaagagc 240
   cacgatcacg aattctggat cg 262
<210> 104
   <211> 287
   <212> DNA
   <213> artificial sequence
<220>
   <221> misc_feature
   <222> (1)..(287)
   <223> probe
<400> 104
   cgttggctct tcatggggat gatggtcagc accacgcctg tcgcggcgct caaaagatag 60
   ctgtggccga gcatgacggg aaacatgctg cgatcctgtg cgacacggcg gatcagcgat 120
   tcctgcgaac cgataccgca gatctggacg ccaagattgg tgacggccgt attgaggccg 180
   aaatagccaa aggatctgaa ttcgtcgaca aggcttctcg aggcctaggc tagggctcta 240
   ggaccacacg tggtgggggg cccagctcgc ggcgcacaat tcactgc 287
<210> 105
   <211> 290
   <212> DNA
   <213> artificial sequence
<220>
   <221> misc_feature
   <222> (1)..(290)
   <223> probe
<400> 105
   cgttggctct tcatggggat gatctgccgg cctgaggggc tgcgcgcacg gaggaaagat 60
   aaggctcgta ggtcatggcc gcgtcgttct ggccggcgat gaaggcctga gcggcaggac 120
   ccggctccat gttgacgacg gtcacgtcct tcacggagag accgttcttc ttcagcatcc 180
   aggagagggc gaaatagggc gacgtgccgg gcgcggaggc cgccacctgc tggcccttga 240
   tgtccttgat ggaggccgag atagccaaag gatctgaatt cgtcgacaag 290
<210> 106
   <211> 285
   <212> DNA
   <213> artificial sequence
<220>
   <221> misc_feature
   <222> (1)..(285)
   <223> probe
<400> 106
   ttgctggcgc cgtcctggtt ggttgcacat tgccattacc cattacgatg gtaatcatca 60
   ccgcgatagc gcaaattgca ccgcctcctg cggctgtttt tcccttcata aagacctcat 120
   aagcgaattt ttacgctcca ggacaaacac ccattcacag ccaataccga ctgactcatc 180
   cctttagaag acacaggata atgcaaatca cttgttagct acgtttcaag atatacatta 240
   ttgctctaat taattatttt tattagggat agataggtgg accat 285
<210> 107
   <211> 271
   <212> DNA
   <213> artificial sequence
<220>
   <221> misc_feature
   <222> (1) .. (271)
   <223> probe
<400> 107
   ttgctcgcgc cgtcctggtt tgcgagacgc aggaggcaag tcgcagtacc agtcgtagaa 60
   gcttaagcaa gtaccgccaa tcagcgagag atagcgtgca cccgatgcgt aagaaaccat 120
   cgacattgcc ggaattggcg agaaccagca acacggtccg ggccgctagt ttttgatggt 180
   gtaacgttag atgcggcgat cagttcgttc acctcctgcc aggagaacga acaatccacc 240
   gccgtcacgc gcctgcttaa ggctttgcgc t 271
<210> 108
   <211> 269
   <212> DNA
   <213> artificial sequence
<220>
   <221> misc_feature
   <222> (1)..(269)
   <223> probe
<400> 108
   aaggagaccg aagaggaaca tgggctggaa gaagatggaa aagccaaaag gaacctttta 60
   cgtatgtggc gtgtagaatt ccgagaaacg tttgagaaca tctcaccaat tctccgatta 120
   cttgctggag catgctcatg tcgttgtcac accgggcgaa aatattcgga agcacggaaa 180
   aaggcatgtc agaatatcga tggtgtcgaa gcaggaggat ctgcgggaat ttgtcatgcg 240
   gattcaaagc tgaacctgct cgtggtgcg 269
<210> 109
   <211> 281
   <212> DNA
   <213> artificial sequence
<220>
   <221> misc_feature
   <222> (1)..(281)
   <223> probe
<400> 109
   tgaaggagac cgcaggagga acacggaaat aagaactgat gtgctcgcag gaaataaaga 60
   cacagggaaa tatgatcata gatactcaaa cattccttaa ctatagggag cagagcgagg 120
   cattaaaggc ctggcagaaa tcaaatccta aggaaggtga atcattacca actatttcaa 180
   caatatcaga attgaataag aaaaaatata ttttgagaaa ttgccacaaa aagctgtcta 240
   ttttggacag cttttataaa ctactgaact gctagtggtg c 281
<210> 110
   <211> 457
   <212> DNA
   <213> artificial sequence
<220>
   <221> misc_feature
   <222> (1)..(457)
   <223> probe
<400> 110
   ttgcgcacga cgatggagaa ctgatttcgc tgtcctcgat ccggcagtcc tcggagacgg 60
   acgtgaacgg accgatgtag gcgtcgttga ccaccgtgcc ggcaccgatg atggcaggcc 120
   cgacgatgcg gctgcacact gacgctggcg ccgcctcgac ccggacccgg ccgatgatct 180
   cgctgctctc gtcgaccgtg ccctcgacca ccggctccga cggtcctcca aggaccgacc 240
   ggtggacctc caagcatgtc gggtaacgtt gccggtgtcc ttccaggtag ccgagagaaa 300
   ggtccgttgg aggcgaacgt acacggctgg ctgggtcaat acagcacact gtgaatggcg 360
   tggtgggtga aaattctatc aggctcggcc ggcgcacaga gaccggctca tatatagacg 420
   caggacggcg ctcttggtga attgccggtg ataaaaa 457
<210> 111
   <211> 302
   <212> DNA
   <213> artificial sequence
<220>
   <221> misc feature
   <222> (1) .. (302)
   <223> probe
<400> 111
   caaagcggaa gcgatgcgga tgttgcgggc aaaagtcgcg gccttagcgg cgcaacaggc 60
   tgctgatgaa aatgatcgaa tggcgtcgag gaccagggtg gcgtactggt cggataggat 120
   cgggctcgag gtggcgtacc ctataacttt cccgaggaat cgcctggacg ggatcatcgt 180
   aattgggtac aagttccagg aacttgacca gagttctggc tggcggacct aggtggatgg 240
   tctaggacgc ggctccatgc cgataggtgg agggcgtgga tggcacaacg gccgaaggtc 300
   ag 302
<210> 112
   <211> 268
   <212> DNA
   <213> artificial sequence
<220>
   <221> misc_feature
   <222> (1)..(268)
   <223> probe
<400> 112
   tgcgcacgac gatggagaaa gccggctata tggacgaaga tttcttccta tatgccgaag 60
   aagtggagtg gtgcagccgt ttacgtaagc tgggcgaatt agcgatcttt ggagacatca 120
   acattattca ccttcagggt gagaccaccg gagacgcctt tgactcagcc gataaggcta 180
   ctacggcctg tatgaccgta aaggcctcca gctcatgtta tccaatcatg tcagggtcag 240
   aaacaattcg gggcacgctg gtacttat 268
<210> 113
   <211> 276
   <212> DNA
   <213> artificial sequence
<220>
   <221> misc_feature
   <222> (1) .. (276)
   <223> probe
<400> 113
   tcgcgcacga cgatggagaa ttgggtatcg tgaaggtata attgaggagg agtataaagt 60
   accagcgtgc cccgaattgt ttcctgaccc tgacatgatt ggataacatg agctggaggc 120
   ctttacggtc atacagggcc gtagtgagcc ttatcggcgt gagtcaaagg gcgtctccgg 180
   gtgggtgctc acccgggggg tgagtgatgg tggatgtgcg ccaaagagtt cgggctaatt 240
   gggggcagcg ttacggtgga acgggctgcg aggcac 276
<210> 114
   <211> 281
   <212> DNA
   <213> artificial sequence
<220>
   <221> misc_feature
   <222> (1) .. (281)
   <223> probe
<400> 114
   cgtcgtttga gcggacatgc gctaaaggca gtgaaattat ccgcctggtt gaagaaagcg 60
   atccggtagc ggaactggca ttgcgtcgct acgagctgcg gctggcaaaa tcgctggcac 120
   atgtcgtgaa tattctcgat ccggatgtga ttgtcctggg gggcgggatg agcaatgtag 180
   accgtttata tcaaacggtt gggcagttga ttaacaattt ggtcttcggc ggcgatgtga 240
   acgccggtgc gtaggcgacg acgggtgaat cacgagttct g 281
<210> 115
   <211> 286
   <212> DNA
   <213> artificial sequence
<220>
   <221> misc_feature
   <222> (1) .. (286)
   <223> probe
<400> 115
   gcttgcgggt ggagatgatc ttggcctcgg ggatcgttca tcgccaggat caccggcctt 60
   ggacgtcggt tcatttccag gctctggcca ggaacatctg ggtcttcggc gtcggcgaac 120
   aggatgcggc ggcctcggcg gtattgcgct cgacatcacc gggtcggagt cggggctgac 180
   caggcgatag cctttggcac ttcaggtggg tctaggcggc cgggccggtg gcgggccatg 240
   cccatgatca ggatctgcgc atcgccagcg accaccggtt gctcgt 286
<210> 116
   <211> 262
   <212> DNA
   <213> artificial sequence
<220>
   <221> misc_feature
   <222> (1) .. (262)
   <223> probe
<400> 116
   cgcagcacga tggagaagtg tggaacgtct gctctacaat gcctttacgg gcatcgatca 60
   ggatcaggga aacctgcgca ttggaagcac cggttaccat gttacgggta tattcgatgt 120
   gacctggtgt atctgcaata atgtatttac ggctgggggt ggaaagtaga tatggccaca 180
   tcaatggtga tacctgttca cgttcagcca caaggccgtc tgtcagcaat gacaggtctg 240
   taaaatcaag tcctttgcgt tg 262
<210> 117
   <211> 279
   <212> DNA
   <213> artificial sequence
<220>
   <221> misc_feature
   <222> (1) .. (279)
   <223> probe
<400> 117
   cgcgcacgac gatggagaat cgatcgggtc cgccttcaac gatctgttga ttggcagcac 60
   agtctcgaac cggctcgaag gtgggaacgg caatgacacc ttccgcggca cgcggagcag 120
   acgtattgat cggtggtgac ggcacgggcg acacggcaga ctattcagcg tcctcggccg 180
   gcatcctggt cacgttgact gccatctcca acggagcaac agacagggtg ccgggggggg 240
   accgaactta gagtgttcta atgcgagcta gagccatgt 279
<210> 118
   <211> 288
   <212> DNA
   <213> artificial sequence
<220>
   <221> misc_feature
   <222> (1)..(288)
   <223> probe
<400> 118
   tcggtcggga cgtgctccac acgcgagcaa caactaccag gacaagcacc aggccctgtc 60
   ccgctatgcg aacgtgatga cgtgcagccg caccaaggtg ccctggcgcc cgggccgcgg 120
   ctacaacagc agcgaaccga agatctacgg cttgcagacc gccacgtggt cggcccggcg 180
   gcgaggaaat ctacaccgac gaatatggcc gggtgcgcgt gcagttccac tgggaccggg 240
   agggcgcgaa cgacgagcgc agggtcagcc tggataccgc gtccgcac 288
<210> 119
   <211> 289
   <212> DNA
   <213> artificial sequence
<220>
   <221> misc_feature
   <222> (1)..(289)
   <223> probe
<400> 119
   ccgcgttcgt gcggaccaac tggcgatata gaacgggcca gggcaggccg ttggctgcga 60
   aaatagggcc tggtcctatc ggcgggctgg atctccaggg tgcgcatcct gatgaggctg 120
   agagttggca gggtagcccg gctgcgacca ggcagggtga ccgggtcggc gagcatttcc 180
   attgatacag ttgctctggt gagcagggct tttccagggt cgtcctgcgt ccaggtgtcc 240
   gacgggtgat gggatggagc cagctgggaa ggactggtga gccactctg 289
<210> 120
   <211> 298
   <212> DNA
   <213> artificial sequence
<220>
   <221> misc_feature
   <222> (1)..(298)
   <223> probe
<400> 120
   catggataac gcctggcagg gaacacctac tgtccaacgt cggtctgttc cgaagggtgg 60
   tgtcaatcag gtgggtggat cagagtgggc tacaaggtcc ttccagctgg ggtcatccca 120
   ttaccgggtc ggacactggg agcaggacga cctggaaaag ccctgctaca ggagcaactg 180
   tgatcaatgg acatgcttgg ctgaccggtc accctggctg ggtcgagccg gctacctgcc 240
   aatctcagcc tcatcaggag tgcgacctgg gagatcaagg cggccatagg acaggcca 298
<210> 121
   <211> 296
   <212> DNA
   <213> artificial sequence
<220>
   <221> misc_feature
   <222> (1) .. (296)
   <223> probe
<400> 121
   cactacgtcc gcgaccctcc tgaagtcggc agcaatcttt tccagcccgc ccagcgacat 60
   ttcattttgc tgcgcgatat aggcgtcata cagagccatt tgctcgttgt atttcgctat 120
   ctgtgcatct gttggttcat ccggtaactc tttcggcggg tttaaccgct ttcagtttct 180
   tacggtttta cctgcctcgg caaaccgtct gagcattcag gatccccacc tttgaagggt 240
   caaggttaag gggcattgca gataatgcgc ttgagcttct ggtgctgcgt ttttta 296
<210> 122
   <211> 300
   <212> DNA
   <213> artificial sequence
<220>
   <221> misc_feature
   <222> (1)..(300)
   <223> probe
<400> 122
   cggtgttcgt gcggaccaaa aggaacgaat ttcagacatc agcacccaac tgaacgcctt 60
   tcccggctgt gaagttgctg tcagcgacgc gccgagcggt ccagttgatt gtggtggtgg 120
   aagcagaaga cagcgaaacg ctgatccaaa ccattgagtc agtacgcaag tagagggcgt 180
   gctggcggtg tcgctgggtt atcaccagca ggaagagcaa ggtgaggaaa caccatgaaa 240
   ctcagtcgtc gtagctttat gaagctacgc cgttgcggcg ctgcggcgcg tgccggtctc 300
<210> 123
   <211> 271
   <212> DNA
   <213> artificial sequence
<220>
   <221> misc_feature
   <222> (1)..(271)
   <223> probe
<400> 123
   cggtccggga cgtgctccag acgctgcgcg accgcgaata tgtgaagacc gaaaagaagc 60
   ggctcgtccc cgaggacaaa ggccggatcg tcaccgcctt cctggagagc ttcttccgcc 120
   gctacgtgga atacgacttc acggcggatc tggaggagca gctcgaccgc atctccaatt 180
   ccgagatcga ctgggaagca ggtgcttcgc gatttctggc gcgacttctc ggcagccatc 240
   ggcgagacga agagctgccg caccgcggag t 271
<210> 124
   <211> 256
   <212> DNA
   <213> artificial sequence
<220>
   <221> misc_feature
   <222> (1) .. (256)
   <223> probe
<400> 124
   cggtcgtgga cgtgctccag gcgacctcgt ccaggctgag gctgatttca tcgagccagg 60
   cgagatagca gttgaggtcg tcggtgtagg tggcgatcgt gcccaccgac gcccccagct 120
   ccgtgggcca gggcatcgag ccagaggtgg gctaatcgct gattggtccc acgaagacca 180
   gcgttcgtgc ggaccaacag gggccgtact cctgtattct ttcagaagga tctggggaag 240
   actcgaactt gctgga 256
<210> 125
   <211> 282
   <212> DNA
   <213> artificial sequence
<220>
   <221> misc_feature
   <222> (1) .. (282)
   <223> probe
<400> 125
   ccagaattcg tgatcggtgt tcgtcggacc aatcccgctg atcacctcga cctcacgcat 60
   aggatcggat caggtgctga tctcgcaaac ccttaggacc tgtcgtcaga gcgaagggga 120
   gggggactgt tattccacca tctctgtgtc gaactcggcc agagtgctcc gcgctgtgat 180
   cagatcctcc aggcttctca atcgggcgat aaggcgatcc agccgcggtg tgagaaagat 240
   caggtagcgg cttggttctc cgacctgtag tgatgcgcca gc 282
<210> 126
   <211> 287
   <212> DNA
   <213> artificial sequence
<220>
   <221> misc_feature
   <222> (1) .. (287)
   <223> probe
<400> 126
   cggtcgggga cgtgctccag gaaatgaact gatccttctc cggcttgccg cgggcctgct 60
   gatagtagcg gatgaagcgc acgctggaat cgaccgcgtc cgatccgccc agggtgaaat 120
   agatgtggtt gagatcgccc ggcgcccgct cgggctagtg ccgaggcagg cggatgcccg 180
   gctccgcgcc gaggccgaaa tagccggtcg cataaggcag ctcccgcatc tggcggctgg 240
   cggcttccac gagtgctggt catgggccgt agccgggcgt tgacgcg 287
<210> 127
   <211> 413
   <212> DNA
   <213> artificial sequence
<220>
   <221> misc_feature
   <222> (1) .. (413)
   <223> probe
<400> 127
   gcgttccggg ctcgaaggaa acgttcagaa ggtcagctat atcggccggc gattctttgc 60
   ttcgtacctg cgcgacggcc gcaccgaagt aaggatgtac gatgaggcgc agagtctggg 120
   cgtcgtaccc tgccgggtct cgggcgcgct gtcggttttg aaggacggaa acgagacgga 180
   acgttctacg tattcacaag ctacacggtt ccctccgttg tttaccacta cgatttaaag 240
   accacaagag cactcttgga agcaaccgaa ggtcgacgcg gatctacgaa atatgagacc 300
   agcctcgtct tctacaacac aaagatggca cgcgcgtacc attgttcatc accgcgcgca 360
   aggatataag ctggacggaa cagaatcccc tttaccctat gatacggcgg atc 413
<210> 128
   <211> 300
   <212> DNA
   <213> artificial sequence
<220>
   <221> misc_feature
   <222> (1)..(300)
   <223> probe
<400> 128
   gcgttccggg ttcgaaggaa gcaacttcca gcaggcggaa cgcctcatcc ctggcatcgc 60
   atttcgctga tatcgttcaa ccgttcaacg cgcacgttgg taatttccaa cagaatgcgt 120
   gatgcccatc gcggcatgtg aattgatgga cgccacccac catcaaactt tcattcacag 180
   gtgtgaggtt tccaggtcgg gcatcatcgg gtatcgacca taaggccgta atcaccaggg 240
   tttttggtcg ggaactgggc cgaataaatc cttgctgcgg ttcttctcat ctgccacgac 300
<210> 129
   <211> 290
   <212> DNA
   <213> artificial sequence
<220>
   <221> misc_feature
   <222> (1)..(290)
   <223> probe
<400> 129
   gcgttccggg ttcgaaggaa ggcttggact taatgagcaa ggagcggagg taatcgaaat 60
   ggcaccattt ccaatcgaaa cgatactggg gaaagccggc gccctctctg tcttcctgtt 120
   catcggagtc gcctttggat gggtgttgga gaacgccgga ttcggcaact caccaagctg 180
   gcagcacagt tttatttcag agagatgacc gttctcaagg tcatgttcac ggccatcgtc 240
   gtcgccatgg tcttgatatt cgcgacttca ggtctggggc ttctagacta 290
<210> 130
   <211> 264
   <212> DNA
   <213> artificial sequence
<220>
   <221> misc_feature
   <222> (1)..(264)
   <223> probe
<400> 130
   gcgttccggg ctcgaaggaa tactgtctca tgaacaggat atgctgcgtc ttcgcatcat
   60
   gatctggcgc actcttgcga ccgacacctt tgacatcgct ctgccggtta accagtcctt
   120
   tgatgtatgg gcaaccatca ttcgtggcaa attccagact gtatatcgcg acattattag
   180
   cgcgttaaat cttctggtgc gatggggatg tttgctggtg ctgatgcagc atctttcttc
   240
   aaacagttgc cgaaggattt cttc
   264
<210> 131
   <211> 273
   <212> DNA
   <213> artificial sequence
<220>
   <221> misc_feature
   <222> (1)..(273)
   <223> probe
<400> 131
   ggcggttccg ggatcgaagg aaccgttcag aaggtcagct atatcggccg gcgattcttt
   60
   gcttcgtacc tgcgcgacgg ccgcaccgaa gtaaggatgt acgatgaggc cggcaagagt
   120
   ctgggcgtcg tacctctgcc gggtctcggg cgcgctgtcg gttttgaagg agggaagacg
   180
   agacggaaac gttctacgta ttcacaaggc tacacgggtc cctccggtgg tttaccacta
   240
   cgagttaaag acccacagga gcactccttg gga
   273
<210> 132
   <211> 261
   <212> DNA
   <213> artificial sequence
<220>
   <221> misc_feature
   <222> (1) .. (261)
   <223> probe
<400> 132
   cgtagagatg gggtctctcc atgtgcccag gctgttatcg aactcctggg ctcaagtgat 60
   ccttctgcct tggtctccca aagtgctagg gttaaaagtg ctggggttat aagtgtgagc 120
   cactgcctct agcccagttt tttagttctt gttacaaatt gccaagtaag gactaatcca 180
   aaagactgga gtattttgtc aatgaacatg tttcaacata tgtatctctt acaaaatgca 240
   gctggtttaa atcctaaagg c 261
<210> 133
   <211> 285
   <212> DNA
   <213> artificial sequence
<220>
   <221> misc_feature
   <222> (1)..(285)
   <223> probe
<400> 133
   cagcgcggca gtgggtgggt tattgctctg ttagctgtgc tggtactggt tggagccggg 60
   gtgttcttct acgtcaaggg gatgcccgga tctcattcgg atgccgctcc tcaaccaacc 120
   caggcaccaa tctctacctc tacgccagag gtcaggccaa cgcgaactgt gacgctcatg 180
   ccacggtgac aacgatgagt tctcccatac agatccagct tcctggcggg gcggtggagt 240
   gtggacaagg ggccttgatc gcaaatcctc gcaccacctg tctct 285
<210> 134
   <211> 280
   <212> DNA
   <213> artificial sequence
<220>
   <221> misc_feature
   <222> (1)..(280)
   <223> probe
<400> 134
   gttcagcggg ttggcgttca gaagcagcgt ggctgggtgc cggatggtgc gatccacgcg 60
   atcgccgatg tgctgggtat tccggcaagc gacgtcgaag gtgtggcacg ttctacagtc 120
   agatcttccg ccagccggtt ggtcgccatg tgaatccgtt attgtgacaa gcgtgtctgt 180
   catatcacgg tatacaggta atcggcgcgc tcgagaaaag ctgactcacc gggcacgaca 240
   tttgataggc gcttaagctg ctgccactgc tgctgggact 280
<210> 135
   <2.11> 271
   <212> DNA
   <213> artificial sequence
<220>
   <221> misc_feature
   <222> (1)..(271)
   <223> probe
<400> 135
<210> 136
   <211> 236
   <212> DNA
   <213> artificial sequence
<220>
   <221> misc_feature
   <222> (1)..(236)
   <223> probe
<400> 136
   gttcagcggg ttggcgttca gggattggtg catttgcctg cccttgctgc ctggaaccct 60
   gaaaatcccg gtgactttgg cggtttgggc atgagcagtg acgagtcagc cattttctat 120
   gcaatcggta ttggcgatgg cagctgggga gcattttatg atgtttgctg cctgtacccc 180
   tacgtacggc aatctttggc tttagcagtc atttgcagtt ggtgcatggc cgtgtg 236
<210> 137
   <211> 264
   <212> DNA
   <213> artificial sequence
<220>
   <221> misc_feature
   <222> (1) .. (264)
   <223> probe
<400> 137
   tcccggtccg gtggtcatga tccttcgccc tctgctcacg aaagatgctg tccgcccatc 60
   ggaagaactc actatttcgc ggttgtgttg gtgggatccc ccggagcccg catcgcgcgt 120
   gcgcatgagc tcattcgaga ggtgggcgac gagacttgag aggaaagcgc tggcgccggg 180
   tgatggaagg cacacagtgc tcaacgcgga cgataccgat tggtccatct gtttcgtata 240
   ggtccatgtg cttctcaact acat 264
<210> 138
   <211> 301
   <212> DNA
   <213> artificial sequence
<220>
   <221> misc_feature
   <222> (1)..(301)
   <223> probe
<400> 138
   accgtgtcga aggcgtttaa catgccggtg gatgagttac agggaagtgc agagcgactg
   60
   aagaaacgcc tcgagaatat gggtgagatc aaccctaccg caattgaggc gtacctggaa
   120
   atgaagaaac gttacgaatt catacttgaa acagaaagac ggatcttgga tctggaattc
   180
   gttcggacaa agctttcttc ggagcctagg ctagcttcta gaccacaacg tgtggggggg
   240
   cccgagctcc cggccgcaac aatttcacat tgggccgtcg tttttacaac gcttgttgtc
   300
   a
   301
<210> 139
   <211> 267
   <212> DNA
   <213> artificial sequence
<220>
   <221> misc_feature
   <222> (1) .. (267)
   <223> probe
<400> 139
   tcctggcccg gtcgtcatga tgttcacgtt attatgtagt ctgccggaca ccttattaca
   60
   ggatgagtat cagcagaaga gtgtgaacta tcaggcgcgg tgacatctgt gtggactaca
   120
   gtcagcatac tgactgcgct gtgatggctc tacgatgctc gcgaaaaaca ccccccatac
   180
   catatccgag cgagcgtgat tataacaacg tgcttccgac aagcgagagc ctcgcgctct
   240
   ggatagagat acatcgtgtc agattac
   267
<210> 140
   <211> 293
   <212> DNA
   <213> artificial sequence
<220>
   <221> misc_feature
   <222> (1) .. (293)
   <223> probe
<400> 140
   accctcgaag gcgttcaaca tcgccttcag ccttcattct cagtagttaa tgccatctgg 60
   atggaaaaca gaggaatcta ctgctgtacc gacacatacg acggaggagg tgaatatcgg 120
   cttgaaaatg gcatcgatgc gcggagacaa cagatgcagc aaaggagaaa tgatgtttga 180
   agactactct tgcctgccag ggagagtaca tgccgaaagc agaaggcgta cacatcaaaa 240
   gagatacatg gcgataatac ggaggataca acaggcggga acatgctgtg atg 293
<210> 141
   <211> 251
   <212> DNA
   <213> artificial sequence
<220>
   <221> misc_feature
   <222> (1) .. (251)
   <223> probe
<400> 141
   tcctggtccg gtcgtaatga ttccgagctc gtcagcaatt tcagtactac ggaactgaaa 60
   cttgtcagcc tcatcgggac ctattattat acctattcta cctgcagcct tattgccgga 120
   attggcctgg ataagttcgg tggcaaaaga tcgctttttg caggtgcttt aattctggga 180
   ataggctgtc tgtaatttct ttgcatctcg cttattcagg tgtgtgttgc aggaagattg 240
   ttgcagggag c 251

## Description

### 1. FIELD OF THE INVENTION

This invention relates generally to methods and materials for use in gene cloning. More specifically, the present invention relates to gene probes/primers for use in discovery and characterization of bioactive compound coding genes and gene clusters.

### 2. DESCRIPTION OF RELATED ART

The basic challenges in drug discovery are to identify a lead compound with desirable activity, and to optimize the lead compound to meet criteria required to proceed with further drug development. One common approach to drug discovery involves presenting macromolecules implicated in causing a disease (disease targets) in bioassays in which potential drug candidates are tested for therapeutic activity. Such molecules could be receptors, enzymes or transcription factors.

Another approach involves presenting whole cells or organisms that are representative of the causative agent of the disease. Such agents include bacteria and tumor cell lines.

Traditionally, there are two sources of potential drug candidates: collections of natural products and synthetic chemicals. Identification of lead compounds has been achieved by random screening of such collections which encompass as broad a range of structural types as possible. The recent development of synthetic combinatorial chemical libraries will further increase the number and variety of compounds available for screening. However, the diversity in any synthetic chemical library is limited to human imagination and skills of synthesis.

Random screening of natural products from sources such as terrestrial bacteria, fungi, invertebrates and plants has resulted in the discovery of many important drugs (Franco et al. 1991, Critical Rev Biotechnol 11:193-276; Goodfellow et al. 1989, in "Microbial Products: New Approaches", Cambridge University Press, pp. 343-383; Berdy 1974, Adv Appl Microbiol 18:309-406; Suffness et al. 1988, in Biomedical Importance of Marine Organisms, D. G. Fautin, California Academy of Sciences, pages 151-157). More than 10,000 of these natural products are biologically active and at least 100 of these are currently in use spanning the entire therapeutic spectrum, including antibiotics, anti-cancer agents, and cardiovascular agents, and .also as agrochemicals . The success of this approach of drug discovery depends heavily on how many compounds enter a screening program and how efficiently the screening can be conducted. Thus, indication-specific compound libraries have tremendous advantages to this end.

Typically, pharmaceutical companies screen compound collections containing hundreds of thousands of natural and synthetic compounds. However, the ratio of novel to previously-discovered compounds has diminished with time. In screens for anti-cancer agents, for example, most of the microbial species which are biologically active can yield compounds that are already characterized. This is due partly to the difficulties of consistently and adequately finding, reproducing and supplying novel natural product samples. Since biological diversity is largely due to underlying molecular diversity, there is insufficient biological diversity in the organisms currently selected for random screening, which reduces the probability that novel compounds will be isolated.

Novel bioactivity has consistently been found in various natural sources. See for example, Cragg et al., 1994. (in "Enthnobotany and the search for new drugs" Wiley, Chichester. p178-196). Few of these sources have been explored systematically and thoroughly for novel drug leads. For example, it has been estimated that only 5000 plant species have been studied exhaustively for possible medical use. This is a minor fraction of the estimated total of 250,000-3,000,000 species, most of which grow in the tropics (Abelson 1990, Science 247:513). Moreover, out of the estimated millions of species of marine microorganisms, only a small number have been characterized. Indeed, there is tremendous biodiversity that remains untapped as sources of lead compounds. Conventional methods of compound discovery from these sources is requisite on the successful laboratory culture of the microbial flora, a practice that is only approximately 1 % efficient. Thus, the vast majority of environmental microorganisms cannot be grown in a laboratory and therefore, any potential bioactive compounds that they produce cannot be assayed.

Terrestrial microorganisms, fungi, invertebrates and plants have historically been used as sources of natural products. However, apart from several well-studied groups of organisms, such as the actinomycetes, which have been developed for drug screening and commercial production, reproducibility and production problems still exist. For example, the antitumor agent, TAXOL^{™}, is a constituent of the bark of mature Pacific yew trees, and its supply as a clinical agent has caused concern about damage to the local ecological system. Taxol contains 11 chiral centers with 2048 possible diastereoisomeric forms so that its *de novo* synthesis on a commercial scale seems unlikely (Phillipson, 1994, Trans Royal Soc Trop Med Hyg 88 Supp 1:17-19).

Marine invertebrates are a promising source of novel compounds but there exist major weaknesses in the technology for conducting drug screens and large-scale resupply. For instance, marine invertebrates can be difficult to recollect, and many have seasonal variability in natural product content.

Marine microorganisms are a promising source of novel compounds but there also exist major weaknesses in the technology for conducting drug screens and industrial fermentation with marine microorganisms. For instance, marine microorganisms are difficult to collect, establish and maintain in culture, and many have specialized nutrient requirements. A reliable source of unpolluted seawater is generally essential for fermentation. It is estimated that at least 99% of marine bacteria species do not survive on laboratory media. Furthermore, available commercial fermentation equipment is not optimal for use in saline conditions, or under high pressure.

Certain compounds appear in nature only when specific organisms interact with each other and the environment. Pathogens can alter plant gene expression and trigger synthesis of compounds, such as phytoalexins, that enable the plant to resist attack. For example, the wild tobacco plant Nicotiana sylvestris increases its synthesis of alkaloids when under attack from larvae of Manduca sexta. Likewise fungi can respond to phytoalexins by detoxification or preventing their accumulation. Such metabolites will be missed by traditional high-throughput screens, which do not evaluate a fungus together with its plant host. A dramatic example of the influence of the natural environment on an organism is seen with the poison dart frog. While a lethal dose of the sodium channel agonist alkaloid, batrachotoxin, can be harvested by rubbing the tip of a blow dart across the glandular back of a field specimen, batrachotoxin could not be detected in second generation terrarium-reared frogs (Daly, 1995, Proc. Natl. Acad. Sci. 92:9-13). If only traditional drug screening technologies are applied, potentially valuable molecules such as these can never be discovered. Additionally, plant and vertebrate microbial symbionts can sometimes independently biosynthesize bioactive compounds originally discovered from the host plant. In fact, in many cases (e.g. taxanes), the symbiont population produces a much wider range of related compounds. It is believed that similar biosynthetic pathways exist in both host and symbiont as a result of horizontal gene transfer. Thus, symbiont microorganisms represent a virtually untapped source of novel natural products, but only if new methods are made available that can overcome the limitations of conventional methods in fermentation/culturing and discovery of compounds from environmental microorganisms.

Moreover, a lead compound discovered through random screening rarely becomes a drug, since its potency, selectivity, bioavailability or stability may not be adequate. Typically, a certain quantity of the lead compound is required so that it can be modified structurally to improve its initial activity. However, current methods for synthesis and development of lead compounds from natural sources, especially plants, are relatively inefficient. There are significant obstacles associated with various stages of drug development, such as recollection, growth of the drug-producing organism, dereplication, strain improvement, media improvement, and scale-up production. These problems delay clinical testing of new compounds and affect the economics of using these new sources of drug leads.

At present, the above-mentioned marine, botanical and animal sources of natural products are underused. Currently available methods for producing and screening lead compounds cannot be applied efficiently to these under-explored sources. Unlike some terrestrial bacteria and fungi, these drug-producing organisms are not readily amenable to industrial fermentation technologies. Simultaneously, the pressure for finding novel sources for drugs is intensified by new high-efficiency and high-throughput screening technologies. Therefore, there is a general need for methods of harnessing the genetic resources and chemical diversity of these as yet untapped sources of compounds for the purpose of drug discovery. Discovery through microbial symbionts offers one possibility if methods can be developed that overcome limitations inherent in conventional discovery from environmental microorganisms.

Most recent drug discovery programs have shifted to mechanism-based discovery screens. Once a molecular target is identified (e.g., a hormone receptor involved in regulating the disease), assays are designed to identify and/or synthesize therapeutic agents that interact at a molecular level with the target.

Gene expression libraries are used to identify, investigate and produce the target molecules. Expression cloning has become a conventional method for obtaining the target gene encoding a single protein without knowing the protein's physical properties.

Many proteins identified by screening gene expression libraries prepared from human and mammalian tissues are potential disease targets, e.g., receptors (Simonsen et al. 1994, Trends Pharmacol Sci 15:437-441; Nakayama et al. 1992, Curr Opin Biotechnol 3:497-505; Aruffo, 1991, Curr Opin Biotechnol, 2:735-741), and signal-transducing proteins. See Seed et al., 1987, Proc Natl Acad Sci 84:3365-3369; Yamasaki et al., 1988, Science 241:825-828; and Lin et al., 1992, Cell 68:775-785, (type III TGF-β receptor) for examples of proteins identified by functional expression cloning in mammalian cells.

Once a disease target is identified, the protein target or engineered host cells that express the protein target have been used in biological assays to screen for lead compounds (Luyten et al. 1993, Trends Biotechnol 11:247-54). Thus, within the scheme of drug discovery, the use of gene expression libraries has been largely limited to the identification and production of potential protein disease targets. Only in those instances where the drug is a protein or small peptide, e.g., antibodies, have expression libraries been prepared in order to generate and screen for molecules having the desirable biological activity (Huse et al. 1991, Ciba Foundation Symp 159:91-102).

However, there are other applications of gene expression libraries that are relevant to drug discovery. Gene libraries of microorganisms have been prepared for the purpose of identifying genes involved in biosynthetic pathways that produce medicinally-active metabolites and specialty chemicals. These pathways require multiple proteins (specifically, enzymes), entailing greater complexity than the single proteins used as drug targets. For example, genes encoding pathways of bacterial polyketide synthases (PKSs) were identified by screening gene libraries of the organism (Malpartida et al. 1984, Nature 309:462; Donadio et al. 1991, Science 252:675-679). PKSs catalyze multiple steps of the biosynthesis of polyketides, an important class of therapeutic compounds, and control the structural diversity of the polyketides produced. A host-vector system in Streptomyces has been developed that allows directed mutation and expression of cloned PKS genes (McDaniel et al. 1993, Science 262:1546-1550; Kao et al. 1994, Science 265:509-512). This specific host-vector system has been used to develop more efficient ways of producing polyketides, and to rationally develop novel polyketides (Khosla et al., WO 95/08548).

Another example is the production of the textile dye, indigo, by fermentation in an E. coli host. Two operons containing the genes that encode the multienzyme biosynthetic pathway have been genetically manipulated to improve production of indigo by the foreign E. coli host.(Ensley et al. 1983, Science 222:167-169; Murdock et al. 1993, Bio/Technology 11:381-386). Overall, conventional studies of heterologous expression of genes encoding a metabolic pathway involve cloning, sequence analysis, designed mutations, and rearrangement of specific genes that encode proteins known to be involved in previously characterized metabolic pathways.

WO 95/04150 A discloses the design of two primers added to S. avermitilis DNA to obtain a PCR product.

WO 92/07078 A discloses amplifying L. mesenteroides pUC9 plasmids by PCR using oligonucleotides to isolate fragments of L. mesenteroides genomic DNA.

WO 97/33988 A discloses new mutant forms of human dihydrofolate reductase (DHFR).

WO 94/24277 A discloses a DNA vector which comprises DNA encoding a mutant, antifolate resistant, dihydrofolate reductase inserted into a site within the vector, the presence of which site is not essential for replication of the vector.

WO 99/10539 A discloses a process for identifying clones having a specified activity of interest comprising (a) generating one or more expression libraries derived from nucleic acid directly isolated from the environment ; and (b) screening said libraries utilizing a DNA probe bound to a ligand, wherein said probe is comprised of at least a portion of the coding sequence of a DNA for a known bioactivity.

WO 98/17811 A discloses a mobilizable combinatorial gene expression library comprising a pool of expression constructs, each comprising a shuttle vector (SV) capable of replicating in different species of strains of host cell, the SV containing a cDNA or genomic DNA fragment derived from species of donor organisms, where the cDNA or genomic DNA fragment is operably associated with at least one regulatory region that derives expression of genes encoded by the cDNA or genomic DNA fragment in an appropriate host organism.

WO 96/40968 A discloses a method for preparing a combinatorial polyketide library (CPL) which comprises: (a) providing a population of vectors where the vectors comprise a random assortment of polyketide synthase (PKS) genes, modules, active sites, or portions and at least one control sequence operatively linked to the genes; (b) transforming a population of host cells with the population of vectors, and (c) culturing the population of host cells under conditions whereby the genes in the gene cluster can be transcribed and translated, thereby producing a combinatorial library of polyketides.

WO 96/34112 A discloses a novel drug delivery system for generating and screening molecular diversity.

In view of numerous advances in the understanding of disease mechanisms and identification of drug targets, there is an increasing need for innovative strategies and methods for rapidly identifying lead compounds and channeling them toward clinical testing.

The speed and availability of automated nucleic acid synthesis has led to rapid technological advances in biological research. For example, the availability of synthetic primers for sequencing has permitted researchers to decrease their time and labor involved in sequencing a particular nucleic acid by approximately sixty percent. Another technology which is facilitated by synthetic oligonucleotides is the polymerase chain reaction (PCR). This technique, which involves the exponential amplification of sequences between two synthetic primers, offers unprecedented detection levels and permits genetic manipulation of the amplified sequence. Further, the availability of synthetic primers allows a variety of genetic manipulations to be performed with relatively simple procedures, including site-specific mutagenesis and the custom design of genetic vectors.

Sequences to be cloned are also routinely modified with synthetic oligonucleotides. The modifications of either vector or insert sequence can range from the addition of a simple sequence encoding a restriction enzyme site to more complicated schemes involving modifying the translation product of the cloned sequence with a specific peptide or a variety of peptide sequences. Thus, these technological advances associated with synthetic oligonucleotides has afforded researchers many opportunities to study diverse biological phenomenon in greater detail and with greater speed and accuracy.

Oligonucleotide synthesis proceeds via linear coupling of individual monomers in a stepwise reaction. The reactions are generally performed on a solid phase support by first coupling the 3' end of the first monomer to the support. The second monomer is added to the 5' end of the first monomer in a condensation reaction to yield a dinucleotide coupled to the solid support. At the end of each coupling reaction, the by-products and unreacted, free monomers are washed away so that the starting material for the next round of synthesis is the pure oligonucleotide attached to the support. In this reaction scheme, the stepwise addition of individual monomers to a single, growing end of a oligonucleotide ensures accurate synthesis of the desired sequence. Moreover, unwanted side reactions are eliminated, such as the condensation of two oligonucleotides, resulting in high product yields.

In some instances, it is desired that synthetic oligonucleotides have random nucleotide sequences. This result can be accomplished by adding equal proportions of all four nucleotides in the monomer coupling reactions, leading to the random incorporation of all nucleotides and yields a population of oligonucleotides with random sequences. Since all possible combinations of nucleotide sequences are represented within the population, all possible codon triplets will also be represented. If the objective is ultimately to generate random peptide products, this approach has a severe limitation because the random codons synthesized will bias the amino acids incorporated during translation of the DNA by the cell into polypeptides.

The bias is due to the redundancy of the genetic code. There are four nucleotide monomers which leads to sixty-four possible triplet codons. With only twenty amino acids to specify, many of the amino acids are encoded by multiple codons. Therefore, a population of oligonucleotides synthesized by sequential addition of monomers from a random population will not encode peptides whose amino acid sequence represents all possible combinations of the twenty different amino acids in equal proportions. That is, the frequency of amino acids incorporated into polypeptides will be biased toward those amino acids which are specified by multiple codons.

To alleviate amino acid bias due to the redundancy of the genetic code, the oligonucleotides can be synthesized from nucleotide triplets. Here, a triplet coding for each of the twenty amino acids is synthesized from individual monomers. Once synthesized, the triplets are used in the coupling reactions instead of individual monomers. By mixing equal proportions of the triplets, synthesis of oligonucleotides with random codons can be accomplished. However, the cost of synthesis from such triplets far exceeds that of synthesis from individual monomers because triplets are not commercially available.

It would therefore be useful to develop a method for synthesizing oligonucleotides which are designed for hybridizing to genes coding for bioactive compound coding genes, antibiotics, and secondary metabolites.. The present invention satisfies these needs and provides additional advantages as well.

### SUMMARY OF THE INVENTION

According to the present invention, there is provided a method of targeted cloning and enrichment of genes and gene clusters. This is accomplished by directly cloning the target gene from the source DNA using one of several novel methods presented, for example by creating template derived primers containing target oligonucleotides, adding these template derived primers to a sample of DNA and performing PCR to replicate those genes targeted by the template derived primers. The methods provide the degenerate cloning of the entire family of related target genes from a mixed DNA sample. This collection of related genes is then used to affinity purify and clone larger target gene containing fragments from the sample, representing associated biosynthetic pathway genes. The result is a target gene/pathway enriched genomic library. Also provided are the genes provided by this method and the probes used in connection with this method. These are also useful for hybridization screening of clonal libraries as well as culture collections.

### DESCRIPTION OF THE DRAWINGS

Other advantages of the present invention will be readily appreciated as the same becomes better understood by reference to the following detailed description when considered in connection with the accompanying drawings wherein:

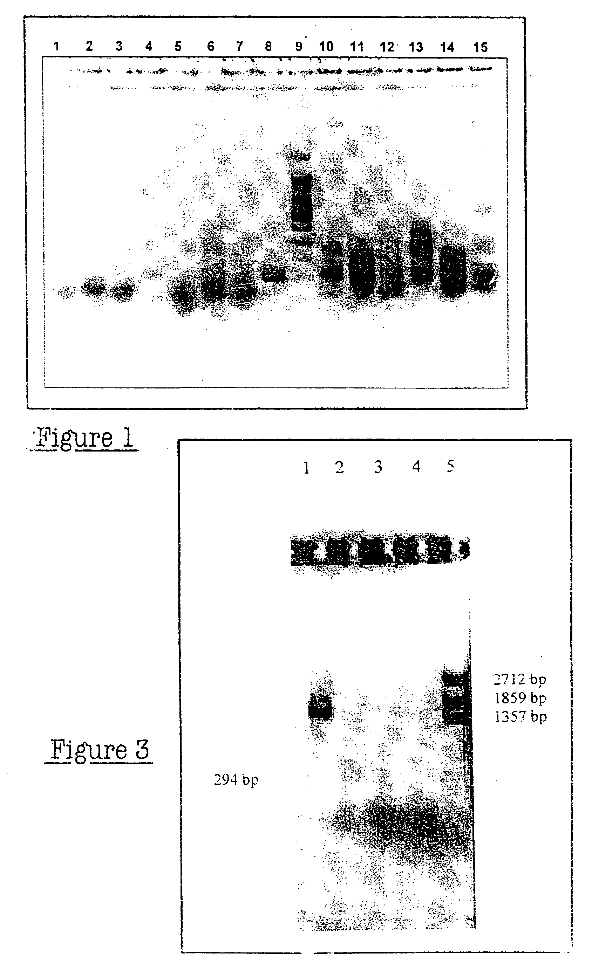

Figure 1 is a photograph showing a gel of the results of the degenerate nested pair PCR reaction for cloning the DHFR2 gene probe from marine sediment DNA; Lanes 10-15 are the products from the first PCR using primers DHFR2-1 and DHFR2-4; Lanes 3-8 are the products from the second PCR using products from the first PCR as template and primers DHFR2-2 and DHFR2-3; Lane 9 contains size markers; Reaction conditions were as specified in the text; The expected product size is about 120 bp as seen in lanes 8 and 4;

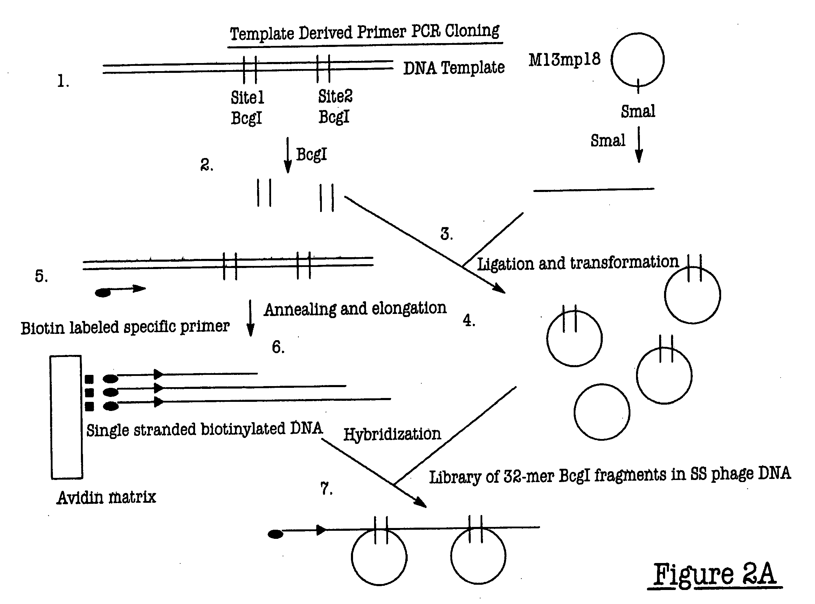

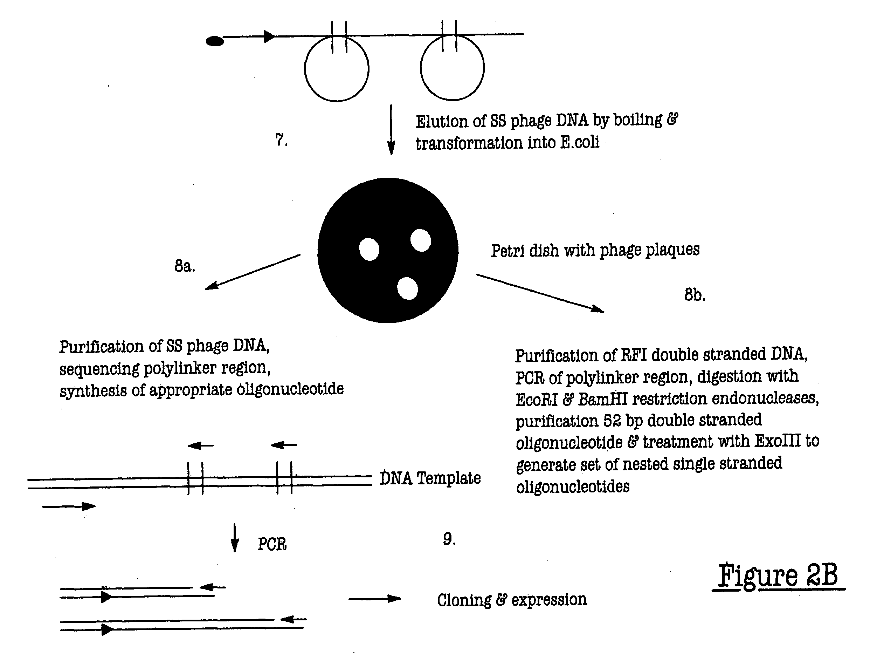

Figures 2 A, B, and C illustrate the strategy for generating template specific primers and their use in specific cloning of unknown flanking sequences against a single known primer, details are discussed in the text;

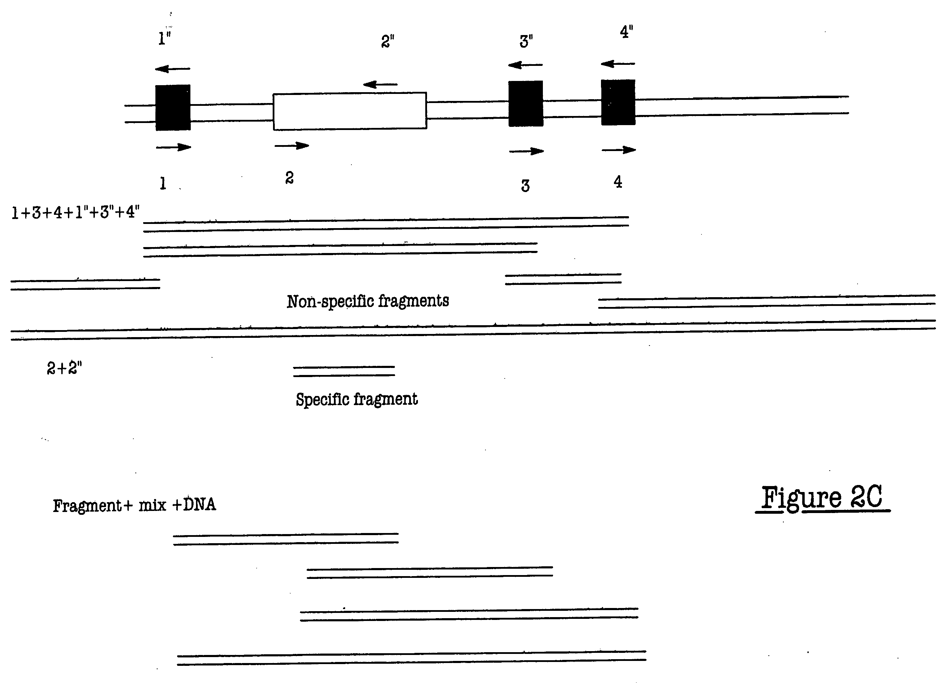

Figure 3 shows the PCR amplification of the part of Amp resistant gene from pBR325 as template using Bcgl derived primers and sequence specific pPstCW primer, the reaction mixture contains pBR325 digested with BamHI (50ng) as template, 32-mer Bcgl primers from pBR325 (gel purified 12pmol) and/or 32-mer sequence-specific primer pPstCW (40pmol); Bcgl primers were denatured five minutes 100 C and immediately cool down on ice; For PCR used program AF08 (T1=96 C, t=30 seconds; T2 56 C, t=1 minutes; T3=72 C, t=10 seconds, reactions are carried out in 34 cycles); Lanes: 1. Mixture of Bcgl oligonucleotide with template; 2. Mixture of Bcgl oligonucleotides plus sequence specific pPstCW primer with template; 3. pPstCW primer with template but without Bcgl primers; 4. pPstCW primer plus Bcgl primers mixture without template; and 5. pBR325 (1ug) digested with Bcgl restriction endonuclease;

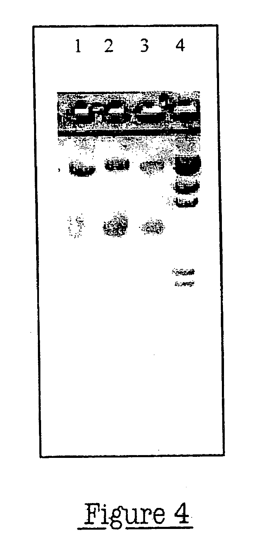

Figure 4 shows the purified single-stranded DNA form from phages run on 0.8% agarose gel (ethidium bromide stained); Lanes: 1. SS DNA M13mp18 (1ug); 2. SS DNA from M13mp18 Bcgl library from *E*. *coli* HB101 (1ug); 3. SS DNA from M13 mp18 Bcgl library from *S*. *clavuligerus* (1ug); and 4. DNA marker lambda DNA digested Hindlll (1ug) (Promega, WI);

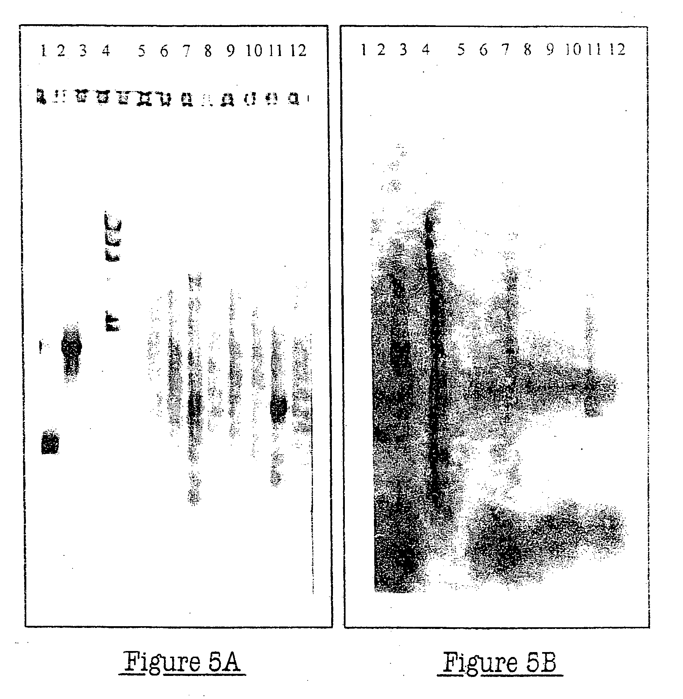

Figure 5 shows a 0.8% agarose gel analysis of biotinylated polymerase elongation products (BEPEP); Panels: A ethidium bromide stained gel. B steptavidin-phosphatase assay from southern blotting. PCR and polymerase elongation reaction ( PER) carried out in 20 ul reaction format contained 30pmol biotinylated primers and 100ng for PCR or 1ug for PER DNA template either CsCl purified DNA from *E. coli* HB101 or DNA from *S*. *clavuligerus;* For improving elongation, reactions carried out with 5u TaKaRa LA DNA polymerase according to the manufacturer's instructions (TaKaRa, Japan); Primers were labeled with photobiotin (Vector, CA) according manual; Lanes: 1. PCR from HB101 DNA, primers AA and AB (sequence-specific primers for PEPase gene of *E*. *coli*), 2. PCR from *S*. *clavuligerus* DNA with primers ACVS 010 and 011 (ACVS 04-011 are sequence-specific primers for ACVS gene of *S. clavuligerus*); 3. Negative control PCR primers AA and AB without template; 4. biotinylated DNA marker lambda digested Hindlll (0.5 ug); 5. Negative control PCR primers ACVS 010 and 011 without template; 6. BPEP with AA primer and DNA HB101; 7. BPEP with AB primer and DNA HB101; 8. BPEP with ACVS 04 primer and DNA *S*. *clavuligerus;* 9. BPEP with ACVS05 and DNA *S*. *clavuligerus;* 10. BPEP with ACVS08 and DNA *S*. *clavuligerus;* 11 BPEP with ACVS 09 and DNA *S*. *clavuligerus;* 12. BPEP with ACVS 010 and DNA *S*. *clavuligerus;* and 13. BPEP with ACVS 011 and DNA *S*. *clavuligerus;*

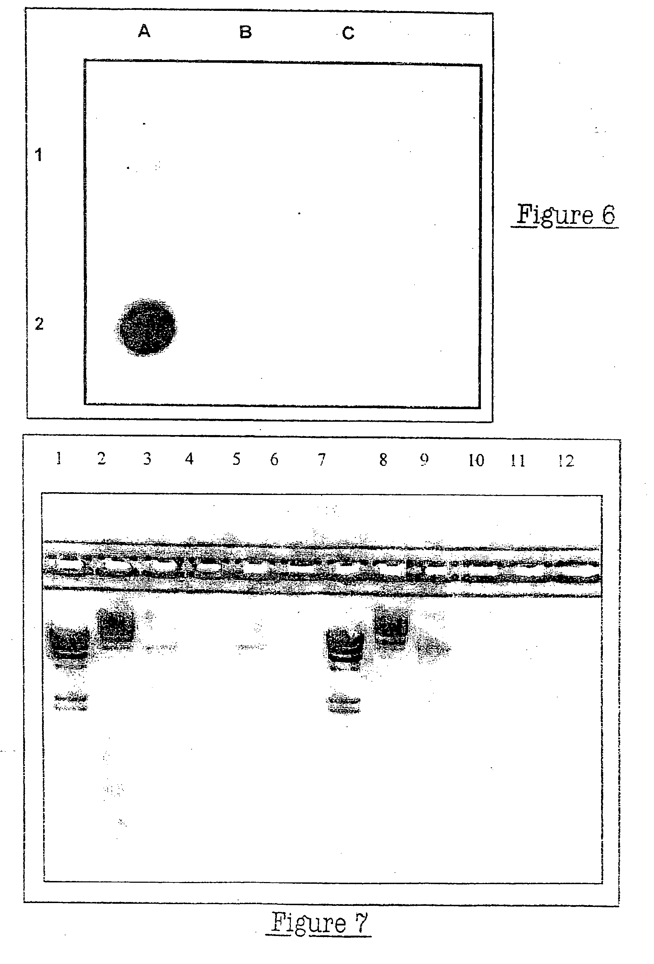

Figure 6 shows a blot of a streptavidin-phosphatase assay of binding of biotinylated polymerase elongated products (BPEP) to Avidin D beads (Vector Labs, CA); Lanes : 1. BPEP with primers AA and AB from DNA *E. coli* HB101 of. 2. BPEP with primers ACVS 04-011 from *S. clavuligerus* DNA. Panels: A BPEP before adsorption to beads, B BPEP fraction unbound to beads, C BPEP fraction incubated at 65 C in TBST buffer; 50 ul (25ng/ul) Avidin beads equilibrated two times with three volumes of TBST buffer and mixed with 100 ul BPEP and incubated 2 hours at 37°C; Then beads washed three times with three volumes of TBST buffer and 2ul analyzed on streptavidin-phosphatase dot blotting assay;
Figure 7 shows a photograph of an agarose gel analysis of bound and unbound fractions of SS DNA form M13mp18 Bcgl libraries; Lanes: 1.,7. DNA marker lambda Hindlll (1ug); 2. M13mp18 original Bcgl library from *E. coli* HB101 (10ug); 3. Unbound fraction of HB101 Bcgl library at 37°C; 4. Bound fraction of Bcgl HB101 library at 37°C; 5. Unbound fraction of Bcgl HB101 library at 65°C; 6. Bound fraction of Bcgl HB101 library at 65°C; 8. M13mp18 original Bcgl library from *S*. *clavuligerus* (10ug); 9. Unbound fraction of Bcgl *S. clavuligerus* library at 37°C; 10. Bound fraction of Bcgl *S. clavuligerus* library at 37°C; 11. Unbound fraction of Bcgl *S. clavuligerus* library at 65°C; 12. Bound fraction of Bcgl *S. clavuligerus* library at 65°C,10 ul (1ug/ul) of M13 mp18 SS DNA library mixed with 50 ul of Avidin D-BPEP (biotinylated polymerase elongated product) in 100 ul of TBST buffer and incubate overnight at 55°C; The temperature was then decreased to 37°C for 10 minutes and 100µl of unbound fraction was collected, The beads were washed three times with three volumes of TBST buffer for five minutes, bound fraction was eluted with 100ul of water by boiling at 100°C for five minutes; All fractions were ethanol precipitated and dissolved in 30 ul water; 10 ul was analyzed by agarose gel electrophoresis and 3ul was electrotransformed into Nova Blue *E. coli* cells (Novagene, WI);

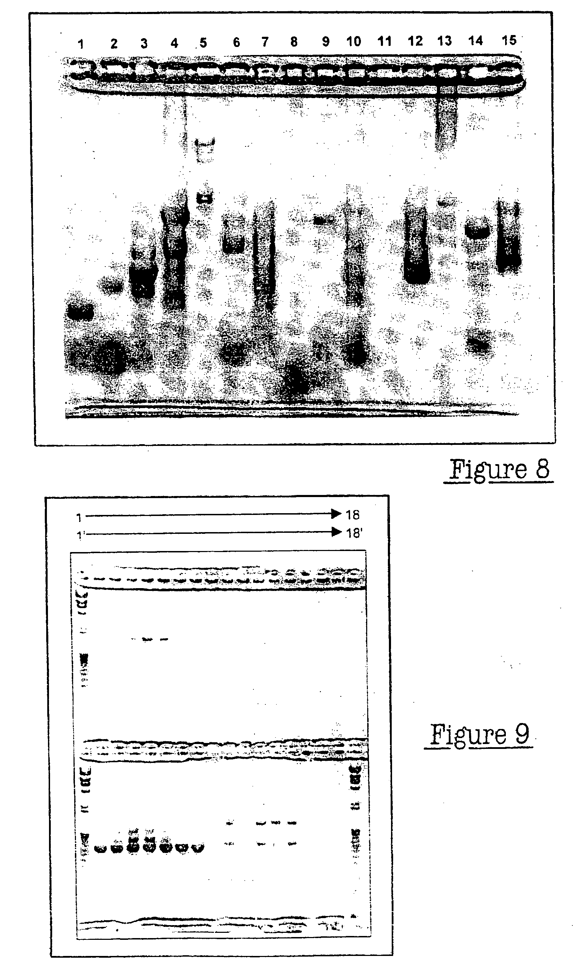

Figure 8 is a photograph of an agarose gel used to analyze the PCR amplification of *S*. *clavuligerus* DNA with sequence-specific ACVS and captured Bcgl primers; PCR reactions carried out in 20 µl format with 100ng of *S*. *clavuligerus* genomic DNA as template and 30 pmol primers; Lanes: 1. ACVS 04 plus 011; 2. ACVS 09 plus 011; 3. ACVS 08 plus 010; 4. ACVS 010 plus 011; 5. DNA marker lambda Hindlll; 6. ACVS 04 plus 04w3bcg; 7. ACVS 04 plus 04w6bcg; 8. ACVS 04 plus 04w9bcg; 9. ACVS 04 plus 04w10bcg; 10. ACVS04 plus 04w13bcg; 11. ACVS09 plus 04w3bcg; 12. ACVS09 plus 04w6bcg; 13. ACVS09 plus 04 w9bcg; 14. ACVS09 plus 04w10bcg.; and 15. ACVS09 plus 04w13bcg;
Figure 9 is a photograph of an agarose gel showing the PCR amplification products of using octamer primers calculated using the k-tuple strategy as described in the text; Template used is HB101 genomic DNA and otherwise standard conditions; Lanes 1, 1', 18' contain size markers; 2-9, oct03 as a solitary primer with varying buffer compositions (lanes 10-18 are empty); 2'-9' standard primers for the phosphoenol pyruvate gene as a control; 10'-18' oct01 as a solitary primer with the same varying buffer as in lanes 2-9 Products are of expected size for a random PCR (0.2-3 kb);

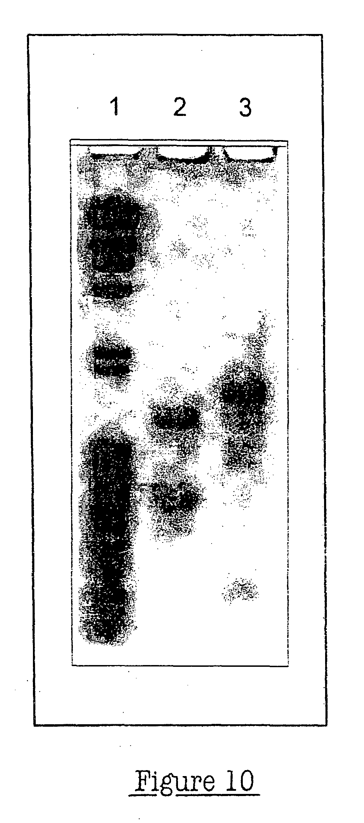

Figure 10 is a photograph of an agarose gel comparing the PCR amplification products from Figure 9; Lanes: 1, markers; 2, oct01; 3 oct03; Reactions were conducted under optimized conditions as judged from analysis of reactions shown in Figure 9;

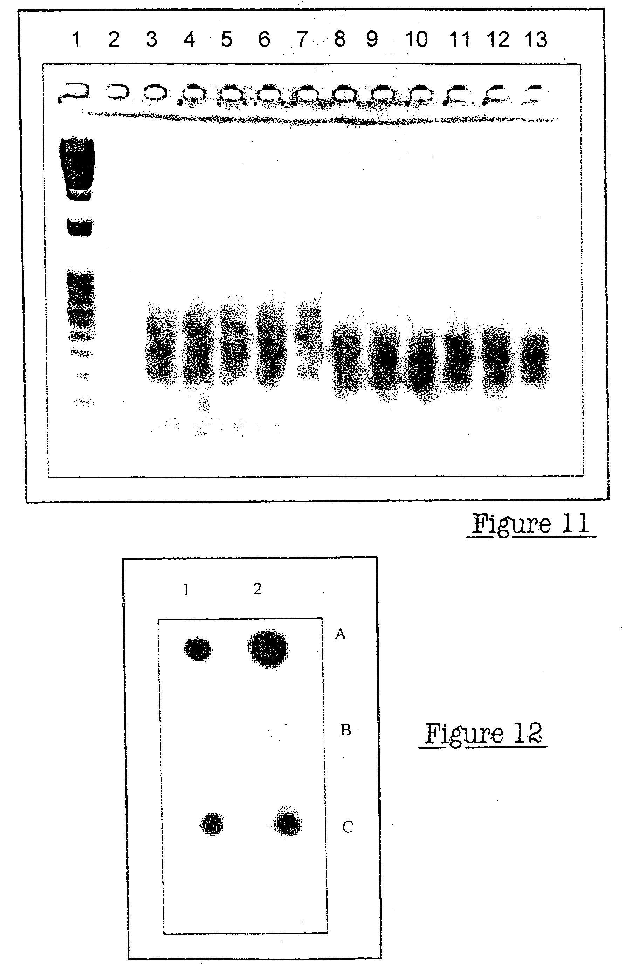

Figure 11 is a photograph of an agarose gel showing the PCR products using k-tuple generated primers pair-wise with primers specific for the *acvs* gene; *S. clavuligerus* genomic DNA was used as template, under otherwise standard cycling conditions and temperature gradient (each primer pair PCR was conducted at 27°C, 34°C, and 42°C, left to right across the gel); Lanes: 1, size markers; 2-4, ACVS05 and oct01; 5-7, ACVS05 and oct02; 8-10 ACVS07 and oct01; 11-13, ACVS07 and oct02; Controls confirmed that amplification was due to pair-wise priming of specific and octamer primers, and not solitary priming by either primer alone;
Figure 12 is a photograph of a hybridization blot analysis of a streptavidin-phosphatase assay of different fractions of biotinylated PCR probes during purification on Avidin DLA beads; Panels: **A)** original mixture of PCR probes 2ul (50ng/ul); **B)** unbound fraction of non-biotinylated PCR probes 2ul (50ng/ul); **C)** biotin eluted fractions of biotinylated PCR probes 2ul (10ng/ul); Lanes: 1. Bio IPNS 05+06 PCR product; 2. Bio StsC03+04 PCR product; 200ul of Avidin DLA beads were used for purification (capacity 25ng/ul);

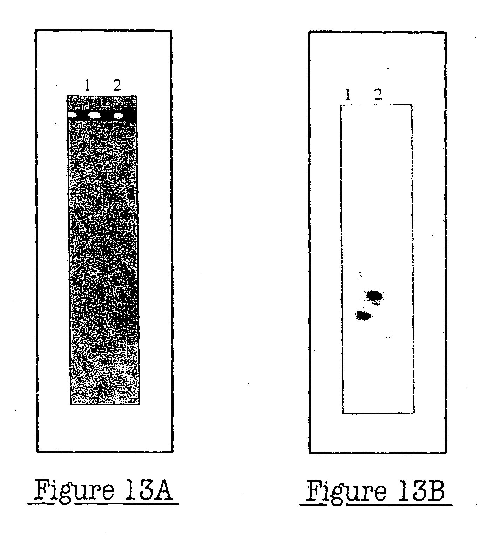

Figure 13 is a photograph showing a 1% agarose gel electrophoresis analysis of Avidin DLA purified biotinylated PCR probes; Lanes: 1. Bio IPNS 05+06 5ul (10ng/ul); 2. Bio StsC 03+04 5ul (10ng/ul); Panels: **A)** ethidium bromide stained gel; **B)** streptavidin-phosphatase assay from the southern blotting of the gel;

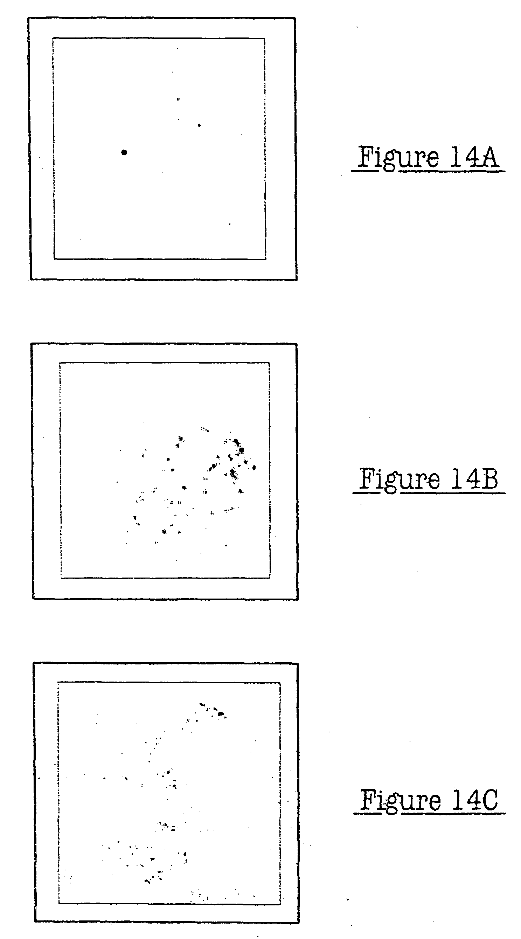

Figure 14 is a photograph of the results of screening pFD666 and pSCOS1 *S*. *griseus* genomic libraries, enriched for aminoglycoside genes, with alk-direct labeled StsC03+04 probes; Panels: **A)** original library (total of 500 colonies on the plate); **B)** StsC and recA captured library after eletrotransformation (total 250 colonies on plate); **C)** library derived from StsC and recA captured chromosomal DNA fragments cloned into pSCOS1 cosmid vector (total 2000 colonies on plate); Results demonstrate over a 100-fold enrichment for the specific gene, as compared to the expected number of positive clones in the unenriched library;

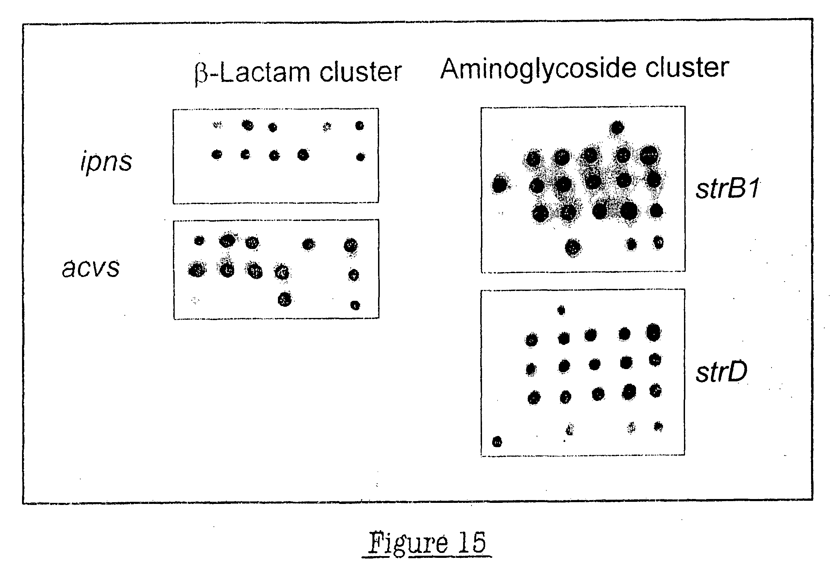

Figure 15 is a photograph of several dot-blots of positive clones from libraries enriched for the acvs gene (left panel) and strB1 (right panel), corresponding to the beta lactam and aminoglycoside biosynthetic clusters, respectively; Genomic libraries were constructed from S. clavuligerus (acvs) and S. griseus (strB1); DNA from positive clones frequently hybridized with several additional gene probes associated with their respective clusters (Table II), demonstrating the cloning of intact clusters and entire pathways;

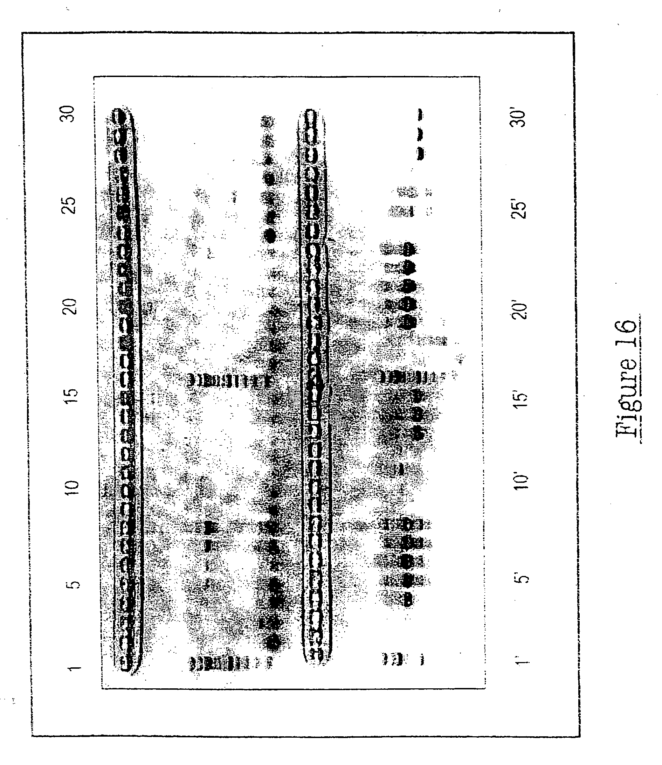

Figure 16 is a photograph of an agarose gel used in the PCR analysis of several clones enriched for the aminoglycoside cluster (Figure 14); Lane 1 to 30: 1^{st} PCR, using *E. coli* cells with different plasmids as template; Lane 1' to 30': 2^{nd} PCR, using 1^{st} PCR products as template; 1: MW marker (100bp ladder); 2 to 8: 1^{st} PCR using StrD primers; 9 to 15: 1^{st} PCR using StsA primers; 16: MW marker (100bp ladder); 17 to 23: 1^{st} PCR using StrB1 primers; 24 to 30: 1^{st} PCR using StsC primers; 1': MW marker (100bp ladder); 2' to 8': 2^{nd} PCR using StrD primers; 9' to 15': 2^{nd} PCR using StsA primers; 16': MW marker (100bp ladder); 17' to 23': 2^{nd} PCR using StrB1 primers; 24' to 30': 2^{nd} PCR using StsC primers, each set of seven lanes with same primer follows the same pattern of order: (1) no template; (2) PDF666 as template; (3) B1-1 as template; (4) -B3-1 as template; (5) B20-2 as template; (6) B20-4 as template; (7) B16str5 as template; These results confirm the retention and stable cloning of the cluster in many clones and corroborates the hybridization results indicating the presence of these genes; Additionally, the utility of many of the oligos listed in Table I and used in double nested PCR as described herein is also demonstrated;

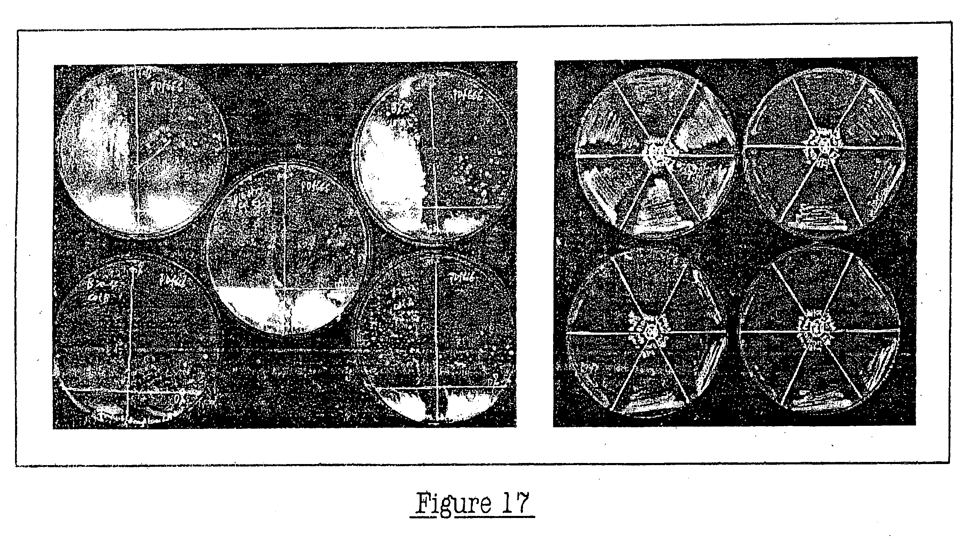

Figure 17 is a photograph of antibiotic selection plates demonstrating the heterologous expression of *S*. *griseus* aminoglycoside resistance in *E*. *coli;* Left Panel: gradient plates from 0 (bottom) to 25 (top) ug/ml streptomycin; The left side of each plate contains a spread from a single positive colony that hybridized to the strB1 gene probe; the right side of each plate contains the *E*. *coli* host transformed with cosmid containing no insert; Right Panel: plates contain the same clones as in the left panel; Plates contain 0, 5, 15, 25 ug/ml streptomycin, clockwise from the upper left plate; and

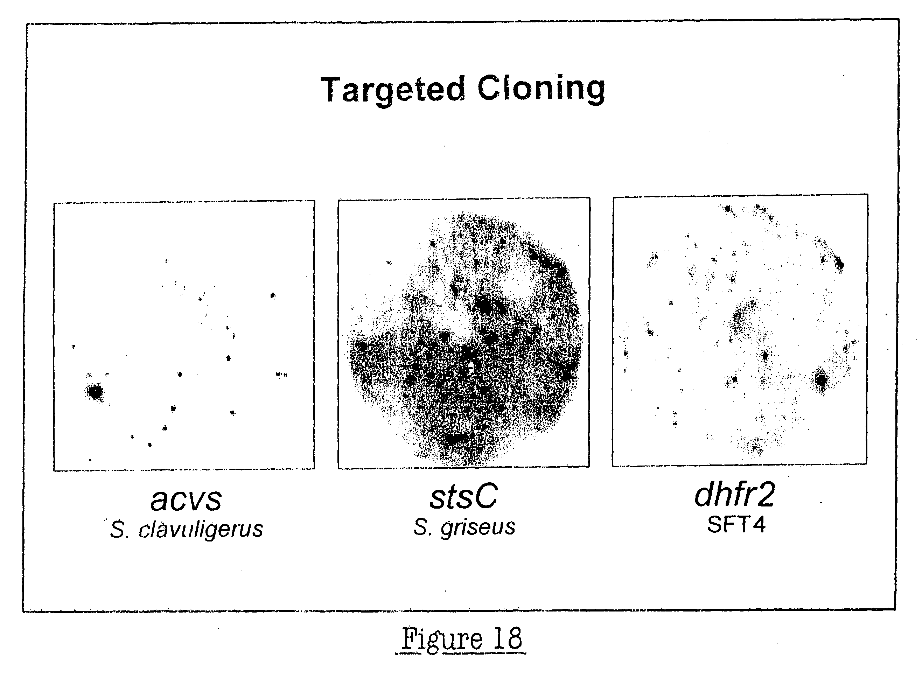

Figure 18 shows yet another example of hybridization probing of genomic libraries using several of the gene probes; SFT4 is a library constructed from a trimethoprim resistant seawater isolate, carries the DHFR2 gene, and demonstrates antibacterial activity against *S*. *aureus* in standard antibiotic challenge assays.

### DETAILED DESCRIPTION OF THE INVENTION

Generally, the present invention provides a method and probes for use in targeted cloning and enrichment of genes and gene clusters from an otherwise mixed and very diverse population of DNA. The methods provide the degenerate cloning of the entire family of related target genes from a mixed DNA sample. This collection of related genes is then used to affinity purify and clone larger target gene containing fragments from the sample, representing associated biosynthetic pathway genes. The result is a target gene/pathway enriched genomic library. Also provided are the genes provided by this method and the probes used in connection with this method. These are also useful for hybridization screening of clonal libraries as well as culture collections.

Genomics and bioinformatics can be used to identify specific genes and DNA sequences that correlate with the biosynthesis of specific structural classes of compounds, including many secondary metabolites. This is often conducted through a comparison of either the nucleotide gene sequences of known related genes or the protein sequences of the gene products through multiple sequence alignments. Constant or conserved regions within related sequences are thought to be important for protein function and will also be conserved in undiscovered genes of the related class. Cloning the entire population of target genes coding for a specific function allows for the associated, clustered biosynthetic pathways to also be cloned in a very specific and targeted manner (see below). Additionally, using degenerate PCR cloning permits the cloning of both closely as well as distantly related genes within a specific target class, subsequently permitting the cloning and capture of the entire genetic and chemical diversity for the target compound class of interest.

Degenerate-nested temperature gradient PCR is used for the successful cloning of the majority or even entire population of related genes from a mixture of many genomes and otherwise unrelated DNA, such as the total DNA isolated from a sample of soil or other environmental source. Nested sets of degenerate PCR primers have been designed for a variety of target genes (see TABLE I).

Several oligonucleotide PCR primers and hybridization probes were designed and then synthesized to target DNA sequences from a variety of sources that potentially contain bioactive compound coding or resistance genes. The design of each oligo was conducted based on the alignment of sequences of the gene and/or protein family of interest that are available publicly (i.e. through GenBank). Several sequences were used, if available. In some cases, only a single unique sequence was available and used in calculating the oligo sequences. Most oligos were designed as degenerate nested pairs in order to maximize their capacity for the cloning and discovery of both closely, as well as distantly related novel sequences that, likewise, code for novel proteins and enzymatic products, such as secondary metabolites useful as lead drug compounds for screening.

The general method used for cloning target genes using degenerate nested temperature gradient PCR uses the following steps. First, a temperature gradient for the 1^{st} PCR is established having a range of temperatures from 41-60°C. This is accomplished using types of buffers having a pH of 8.3-9.2, MgCl₂ (1.5-3.5 mM), KCI (25 & 75mM) (Stratagene PCR Optimization Kit). A volume range between 10-30ul per reaction is placed in a 0.2ml tube for cycles between 30-35. This is ten times diluted and the 1^{st} PCR products are used as templates for a 2^{nd} PCR reaction. The 2^{nd} PCR occurs at 52°C and the other conditions are same with 1^{st} PCR. A gel the then run and expected size of product is cut. DNA is extracted from the gel by using gel extraction kit (Qiagen). The PCR product is cloned into a pT7Blue-3 vector (Novagen), based on manufacturer's protocol. Clones containing the target PCR product are screened by PCR and/or dot-blot hybridization. The plasmid is then purified. Automated sequencing is done using a Thermo Sequenase Cy5.5 terminator cycle sequencing kit (Amersham) and 50-250 fmol template with M13 forward or M13 reverse primers (2 pmol each).

The new sequence is aligned and compared with consensus target genes to confirm the degree of uniqueness by performing a BLAST search and sequence analysis.

In all cases, using these degenerate primers in the following way improves amplification significantly and reduces the number of unrelated misprimed products. This is a problem when it is otherwise desirous to sequence directly PCR products in the discovery of new genes. Most misprimed products can be eliminated by conducting a degenerate, limited-degenerate, nested PCR (DLDN-PCR). The first PCR can be highly degenerate, which aids in the potential discovery of distantly related genes. However, this also results in more unrelated amplification products. The result is clearly seen on an agarose gel of the PCR reaction, where it is seen that the expected product band is rather diffuse, and there is the existence of products of unexpected sizes (Figure 1, lanes 14, 15). Analysis of this band on a 10% acrylamide gel reveals the presence of several bands, each varying slightly in size, and presumably sequence. However, conducting a second PCR using the gel purified product band or first PCR reaction mixture as template and a nested set of less degenerate primers results in amplification of only specific template targets (Figure 1, lanes 4, 8). In this case, a 150 bp product is clearly resolved and amenable for purification and sequencing. This is because the chances for an unrelated misprimed products also containing another mispriming site (in addition to the first which misprimed in the first PCR) is very remote. This was confirmed by cloning and sequencing the PCR products from a single, yet diffuse DHFR2 band of about 150 bp. Although the cloned sequences all contained the priming sites, a much smaller percentage contained any related DHFR2 sequence bounded by the primer sites. Thus, a second PCR results in amplification of only the truly related molecules from the population of products. Cloning and sequencing the products from this second PCR demonstrates this "filtering" effect. The result is a reliable strategy for generating degenerate PCR products amenable for direct sequencing. However, if the number of specific products is high, as is sometimes the case for relatively common amplicons in environmental samples, then cloning the PCR products results in a large number of clones with specific product for sequencing.

Using the above described primers (Table I) and DLDN-PCR, there was discovered and sequenced several unique genes from marine and terrestrial microbial genomes related to a wide variety of biosynthetic pathways and structural classes of compounds, including antimetabolites, beta-lactams, polyketides, other antibiotics, taxanes, and others.

An example of this method involves a SC16RA01 probe which was generated using the "universal" 16S RNA PCR primers to amplify a 600 bp DNA product from S. clavuligerus. This probe is useful for colony hybridization probing for Streptomycetes and other related high GC content genomes. Additionally, this probe has been used in the PCR amplification of similar genomic DNA from a heterogeneous population.

The resulting gene probes can be used for the discovery of either single genes or entire clusters of adjacent genes involved in the total synthesis of compounds of interest, for example secondary metabolite biosynthetic pathways, the products of which comprise very useful libraries for antibiotic and other therapeutic compound screening. This is especially promising since the relatively recently emerging picture of the clustering of secondary metabolite gene pathways on the bacterial and fungal chromosome.

The following adaptation of the present invention describes a method for the generation and use of highly specific PCR primers derived from the template itself. However, their sequence need not be known *a priori.* This adaptation also exploits some unique and novel properties of restriction endonucleases, using Bcgl as an example (Figure 2).

Bcgl is a novel Type II restriction endonuclease originally isolated from *Bacillus coagulans,* and is now commercially available. The recognition sequence for Bcgl is shown in the following and consists of a specific 6 base pair site of DNA sequence. However, the enzyme cleaves outside of this recognition site and generates a 32 bp restriction fragment:
5'-/(N)₁₀CGA(N)₆TGC(N)₁₂/-3'
3'-/(N)₁₂GCT(N)₆ACG(N)₁₀/-5'

Each restriction fragment is statistically unique in sequence and can be used as a specific oligonucleotide primer. The frequency of occurrence of the recognition site is the same as that for a random six base sequence, or about once every 4,000 nucleotides (i.e. (1/4⁶). However, the uniqueness of the fragment is extraordinary because it contains 34 nucleotides and corresponds to a randomized occurrence of once in 2.9 x 10²⁰ bases. Random sequence analysis has confirmed the uniqueness of these restriction fragments and that they are not merely a frequently occurring repeat. This provides the basis for these fragments serving as very specific PCR primers and hybridization probes, each fragment highly specific for its own recognition sequence. By digesting an entire genome, entire or partial chromosome, or mixture of many genomes, very specific primers can be produced with priming sites spaced approximately 4,000 bp apart along the template DNA, ideal for PCR amplification and cloning.

The library of these unique oligonucleotides that are produced from strain specific genomic DNA or a mixed population of environmental DNA can be used as a set of primers for PCR and in combination with gene-specific primers, can be used for amplification and cloning of neighboring regions of DNA surrounding specific genes. Therefore, this technique can also be used for cloning large segments of DNA adjacent to a specific target site, including complete bacterial operons, or biosynthetic pathway gene clusters from any organism. More broadly stated, this adaptation of the present invention can selectively and very efficiently (i.e. with high selectivity) amplify and clone from a mixture of DNA the regions flanking any specific target without any prior knowledge of the sequence to be cloned.

The simplest application of this adaptation of the present invention is to use the entire set of Bcgl template derived oligonucleotide primers in a PCR that also contains a target specific oligonucleotide. A model system has been developed with pBR325. Then, the method of the present invention was used to amplify a 300 bp fragment of the ampicillin resistance gene using a specific primer and a random mixture of template derived primers from a Bcgl digest of the pBR325 plasmid, which contains three Bcgl cleavage sites (Figure 3). It was also determined that this method was effective with both linear and circularized template, using otherwise conventional PCR conditions.

An example of a more extensive and specific application of this adaptation of the present invention involves the identification and isolation from the entire Bcgl restriction digest of the single oligonucleotide containing the priming site most proximal to a specific oligonucleotide on the template to be amplified and cloned. The following steps describe the method of the present invention:
1. DNA isolation and purification from bacterial strains or total environmental DNA from sources such as oil, water, etc. using known procedures such as guanidine thiocyanate, CTAB, cesium chloride gradient or their combination and/or modification;
2. Digestion of isolated DNA with Bcgl endonuclease (NEB, protocol) and preparative purification of Bcgl 34-mer oligonucleotides using 15% PAGE or 2% agarose gel in combination with the QIAEX II purification system (Qiagen, CA) or any similar purification system.
3. Construction of a 32-mer Bcgl oligonucleotide DNA library in M13 phage or any other phagmid vector, that does not contain Bcgl sites. Vector is first digested with Smal, EcoRV or any other blunt-end producing unique restriction endonuclease followed by phosphatase (CIP) treatment. The purified 32-mer Bcgl oligonucleotides are treated with Klenow fragment of DNA polymerase I or T4-DNA polymerase in conjunction with polynucleotide kinase in the absence of any dNTP, but in the presence of ATP in order to convert 3'-protruding ends generated by Bcgl restriction endonuclease to blunt ends appropriate for cloning. The Bcgl restriction fragments are now 32 bp. Equimolar concentrations of the vector and blunt ended 32-mer oligonucleotides are ligated using T4 DNA ligase, followed by transformation into any conventional specific strain of E. *coli* (JM101, TG1 or ER2267) by chemical transformation or electroporation using conventional protocols;
4. The library of phages is washed out from the agar plates following transformation and single stranded DNA is purified by standard methods (Figure 4).
5. Specific primer (specific probe for the gene of interest) is labeled with biotin either at the 5'-end or randomly using a biotin labeling system (Vector Labs), or any other labeling system (e.g. fluorescein) .
6. A single stranded, labeled copy of the sequence to be cloned is produced as follows. Annealing and elongation of the labeled, gene specific primer with genomic DNA template produces a single-stranded, biotinylated copy of the DNA of interest, including sequence downstream and flanking the known region, that which contains the annealing site of the gene specific probe (Figure 5). The biotinylated copy of DNA is isolated by absorption onto an avidin or streptavidin containing matrix, such as Avidex (Vector Labs, CA) or any other affinity matrix (Figure 6);
7. Single stranded oligonucleotide DNA from the phage library (step 4) is then hybridized with single stranded biotinylated DNA under appropriate conditions. All non-specific phase DNA is then washed out and only phase DNA containing complementary sequences will hybridize to the biotinylated DNA. A subsequent boiling procedure releases the single-stranded phase DNA that can then be amplified via retransformation into E. *coli* and amplified *in vivo* (Figure 7).
8. The phage library can be used for the generation of second primers either with PCR of the polylinker region or by Bcgl digestion; Repeating steps 5-7 results in a nested set of PCR primers that can be used to amplify an entire biosynthetic pathway;
9. The phage library is used for generation of second primers for PCR. Of many ways this can be accomplished, two examples were described. First, the 32 bp region of insert was sequenced directly and this sequence was used for oligonucleotide synthesis. As an example, this yielded several primers, including
   GGGTCCGGCAGACCGTTCGCGGGCCGGAC,
   GAGCGGACCGCACCGCGATCGGAACAACCT,
   TCTCCGGGGCAGCGCGGTCGCGGAACGT.

A BLAST search confirmed their relatedness with the genus *Streptomyces,* as expected. Second, the polylinker region containing the 32 bp insert (desired PCR cloning primer) was amplified by PCR using the M13 universal and M13 reverse primers, generating a 184 bp PCR product. The 32 bp Bcg I fragment is flanked with unique EcoRl and BamHl restriction endonucleases sites and restriction with these enzymes was used to generate a 52 bp fragment, which was subsequently converted into a set of nested single-stranded oligonucleotides by treatment with Exolll nuclease under standard conditions. Each oligonucleotide in this nested set has the same 5'-end but a different level of deletion at 3'-end. Therefore, it can be used for PCR cloning as a second primer against a specific target primer. This approach can be used to clone several full length genes, operons, and entire biosynthetic pathways, as demonstrated in the next step.

10. This method was used to clone a region flanking a specific priming site within the *acvs* gene of *S*. *clavuligerus.* Combination of the first primer (gene specific) and secondary primers in a PCR results in the generation of sequences flanking that of the gene specific primer annealing site (FIGURE 8). Additional sequences can be subsequently cloned and combined into one operon for expression of proteins that produce secondary metabolites of interest (for example antibiotics).

Yet another adaptation of the present invention describes a method for the generation and use of highly specific PCR primers with frequently occurring priming sites across a wide range of genomes. These primers are novel and very useful for cloning sequences flanking a target sequence with no prior knowledge of the sequence to be cloned. This set of primers, collectively, is useful as a universal primer library, with specificity based on the criteria used in its generation.

This adaptation of the present invention relies on the analysis and interpretation of DNA sequences from a variety of genera, searching for relatively long and frequently repeated sequences across a wide range of genomes.

As an example, a genomic analysis of bacterial DNA protein coding regions was conducted and subsequently a set of 21 universal priming octamer oligonucleotides was constructed based on a very high frequency of repeating 8 base sequences. In addition, PCR conditions were optimized using various thermopolymerases, including the Stoffel polymerase fragment, and have demonstrated the ability of these "universal primers" to prime against specific target primers. A 10 base universal oligonucleotide primer library was also constructed, and the sequence analysis data reveals that virtually any length oligonucleotide set can be constructed. However, the frequency of occurrence decreases with increasing oligonucleotide length. However, long amplification PCR techniques make even these highly specific but less frequently binding oligonucleotide quite useful.

As an example, octamer and decamer oligonucleotide libraries were generated by performing a k-tuple search and analysis using a proprietary gene database. This database consisted of 15 genera representing 34 bacterial and 4 fungal species, and 38 protein coding genes. The species included in this database were represented in a weighted fashion based on the known/perceived frequency and importance of secondary metabolite production. Of the nearly 65,000 octamers calculated, only a subset of approximately 200, or approximately 0.3%, were frequently present within every or most of the genes included in the database, and thus useful for universal PCR cloning. For example, the octamer OS-OCT-003 with sequence CTCGCCGA occurs 30 times and at least once in nearly every species. This corresponds to a determined average frequency of once every 1,625 nucleotides, while the random frequency for an eight base sequence is only once every 65,000 nucleotides. Similar calculations based on k = 10 resulted in a smaller number of equally frequent 10 base sequences, also useful for PCR primers. An example of 25 octamers and 12 decamers generated and used successfully for cloning are shown in Tables II and III

**TABLE II**

| **High Frequency Bacterial CDS Octamers** | | |
|---|---|---|
| Name | Sequence, 5' -3' | Frequency |
| OS-OCT-001 | GTCGGCGA | 30 |
| OS-OCT-002 | CCAGATCG | 21 |
| OS-OCT-003 | CTCGCCGA | 23 |
| OS-OCT-004 | CGACATCG | 18 |
| OS-OCT-005 | GCCGATCA | 17 |
| OS-OCT-006 | GCCACCGA | 15 |
| OS-OCT-007 | GATGCCGA | 17 |
| OS-OCT-008 | CGGCGAAG | 19 |
| OS-OCT-009 | CGGCGAAC | 19 |
| OS-OCT-010 | GGCGATCA | 15 |
| OS-OCT-011 | GCCGAGGA | 17 |
| OS-OCT-012 | CGCCGACA | 17 |
| OS-OCT-013 | ATCGCCGA | 13 |
| OS-OCT-014 | GGCGAACC | 13 |
| OS-OCT-015 | GCCGACCA | 14 |
| OS-OCT-016 | GCCAAGGA | 15 |
| OS-OCT-017 | CGGCAACG | 16 |
| OS-OCT-018 | GGCTGGAC | 13 |
| OS-OCT-019 | GCAGCACC | 14 |
| OS-OCT-020 | CCAGCCAG | 16 |
| OS-OCT-21 | CGCCGCCG | 39 |
| OS-OCT-22 | CGGCGACC | 34 |
| OS-OCT-23 | CCGCCGCC | 33 |
| OS-OCT-24 | CGCGGCCG | 31 |
| OS-OCT-25 | GTCGGCGA | 30 |

**TABLE III**

| **High Frequency Bacterial CDS Decamers** | | |
|---|---|---|
| **Name** | **Sequence, 5'- 3'** | **Frequency** |
| OS-DEC-001 | CAGCTCGGCG | 8 |
| OS-DEC-002 | GCCGGTGAGC | 7 |
| OS-DEC-003 | CCGGGTCGAG | 7 |
| OS-DEC-004 | GGCGCCGCCC | 6 |
| OS-DEC-005 | GGCGCCGCCC | 6 |
| OS-DEC-006 | CGAGGTCGAG | 6 |
| OS-DEC-007 | CGAGCAGGCC | 6 |
| OS-DEC-008 | CGACGCGGGC | 6 |
| OS-DEC-009 | CCTGGCCGCG | 6 |
| OS-DEC-010 | CCTGCGCGGC | 6 |
| OS-DEC-011 | ACGGCCGCGG | 6 |
| OS-DEC-012 | CGAGGACGTC | 5 |

The specificity of these octamers and decamers toward bacterial protein coding sequences was confirmed by frequency analysis in mammalian DNA. The frequency in human DNA for each octamer was at least ten-fold less than in bacterial DNA, which was used as one criterion for selecting the octamers and decamers from the entire set generated. Additionally, a randomized search against known consensus sequences revealed no matches with most oligonucleotides generated. This confirms that these oligonucleotides are indeed novel, unique, and useful for specific universal cloning of bacterial DNA present in a mixture. Furthermore, both the presence and high-level frequency of several of these octamers were confirmed within several desired cloning sequences (e.g. *S. clavuligerus ipns* gene).

Using this method, PCR with the octamer set has been clearly achieved using *E*. *coli* HB101 genomic DNA (gDNA) as template (Figures 9 and 10). When used as solitary oligonucleotides in PCR reactions, the amplification products were observed in the size range of 0.2 - 3 kb, consistent with that predicted from the calculated frequency of the octamers. This demonstrates the utility of this octamer set for genotyping, in addition to cloning via amplification against a specific primer. A similar result has been demonstrated with *S*. *clavuligerus* gDNA used as template. Additionally, the ability to use these octamers as pair-wise PCR primers was demonstrated by amplifying a product using ACVS-04, a proprietary degenerate primer for the *pcb A* gene (Figure 11).

The present invention is different from random priming and arbitrary priming in the following ways. Random priming is not specific for any type of DNA. Conversely, random primers are generally kingdom specific, as opposed to RAPD, (random amplified polymorphic DNA method) which is a DNA polymorphism analysis system based on the amplification of random DNA segments with single primers of arbitrary nucleotide sequence. Instead, the present invention uses primers specifically designed from thorough analysis of DNA databases, and the resulting oligonucleotides are universal for genomes included in the database. For example, in a method for RAPD PCR differentiation of Streptomyces species, none of the twelve 10-mer oligonucleotides matched the sequence of the over 65,000 oligonucleotides generated by the method for bacterial DNA amplification.

The use of the present invention also has a distinct advantage when the desired target sequence is derived from DNA of a mixed source, for example total purified DNA from soil. This population of total DNA will contain bacterial as well as fungal, plant, and potentially a host of many other contaminating DNAs, making it difficult to amplify specifically a product from a single group of the constituent DNA, such as that of a desired bacterial gene. However, a universal primer set constructed as described in this invention allows for universal priming of a specific subset of the total DNA population, only bacterial DNA in this example. For example, a specific bacterial gene can be amplified from a mixture of bacterial and mammalian DNAs using a single gene specific primer in conjunction with a universal library of oligonucleotides constructed as described in the present invention.

Another example of the utility of the present invention is demonstrated by using it to amplify against a specific primer in order to clone the region of gDNA flanking the specific primer annealing site. *Streptomyces clavuligerus* gDNA was used as template with specific ACVS, IPNS, and other specific primers to demonstrate the technique with high GC containing DNA.

The combined results from all methods described in the present invention for the direct cloning of unique target genes from marine and terrestrial microbial genomes is listed in Table IV. In summary, this collection contains 52 novel genes with homologies with the prototype gene or consensus sequence ranging between 35-90%. This includes a total of 10 classes of target genes, each gene within a class confirmed by sequencing.(See Table IV).

Other adaptations of this invention center around the optimization and refinement of environmental DNA isolation and purification; PCR conditions and additives including DMSO, formamide, and others, use of neutral base substitutions and tails incorporated into the primers (such as d-azaGTP in place of dGTP and inosine tails of 2-6 bases), and specific temperature cycling protocols; the construction and use of degenerate primers based on calculated universal primers, including the use of inosine in primers to increase length and annealing temperature; and the construction and use of labeled primers, such as biotinylation.

Cloned target genes representing the biosynthetic pathways or, in general, any flanking sequence, can be affinity purified from a diverse mixture of DNA, such as environmental DNA or total genomic library DNA. This includes both circular and linear DNA. Subsequently, the entire captured fragment containing the target gene/pathway is cloned and propagated in a variety of expression/cloning host organisms and assayed for bioactivity based on the compound class of probe gene chosen. The method is based on RecA mediated homologous recombination and affinity-chromatography.

Generally, the method consists of the following steps: i) biotinylation and affinity purifying the cloned probe gene; ii) reacting the biotinylated probe with diverse, mixed DNA containing sequences complementary to the probe; iii) capturing the hybrid probe: complementary fragments on an avidin support; iv) eluting the captured fragments; v) and molecular and/or biological cloning of fragments and propagation in any suitable host, such as *E*. *coli* or *S. lividans.*

Other uses of the novel cloned genes include hybridization screening, as exemplified abundantly by the data presented throughout this disclosure. For example, all probes/primers have been labeled with biotin and used successfully for the chemiluminescent discovery of novel target genes from southern blots of environmental DNA and genomic clones. Subsequent cloning and sequencing of these target genes was used to confirm that each probe bound specifically to its intended target. Thus, these probes are very useful (specific and sensitive) for the discovery and isolation of novel target genes, related gene clusters, and biosynthetic pathways.

The use of the DHFR2 oligos is especially promising for the discovery of novel folate antimetabolites, and their coding genes and gene products (biosynthetic enzymes). This approach is the only known source for the DHFR2 genes from which the oligos were generated as TMP resistant clinical isolates. TMP is a synthesized antibiotic and thus a search for a natural producer using genetic determinants for clinical resistance is quite novel (?). The DHFR2 oligo targets a unique form of DHFR protein that it unrelated to the chromosomal or other mutant forms that confer clinical resistance to TMP. Thus, the origin of this gene and protein have not been determined. DHFR2 can originate from a TMP-like biosynthetic pathway, conferring self-resistance to the producer. Following this model, the DHFR2 gene should be clustered within the entire TMP-like pathway. Thus, detection of the DHFR2 gene also provides the entire pathway within the regions directly flanking the gene. The results clearly demonstrate the utility of the method of the present invention have demonstrated the presence and possible origin of this unique gene in several environmental bacterial isolates, as judged by both colony hybridization probing, PCR, and sequence analysis of the gene.

ACVS04 (degenerate) and ACVS05 primers were used to PCR clone and sequence an approximately 500 base pair product from S. clavuligerus genomic DNA. This PCR was designed to generate 400 bp of known S. clavuligerus ACVS and 100 bp of new sequence of this gene. This strategy allows for assessing the accuracy of the sequence by comparison to a known sequence as well as generate new sequence. This confirmation allows for the routine use of the primers for generating new sequence directly from degenerate PCR products, a much more rapid approach than conventionally used.

DHFR2 has been used in the successful discovery and sequencing of several new DHFR genes. These genes confer resistance to TMP and other folate antimetabolites in WT as well as clinical isolates. Additionally, many of the WT strains produce novel folate antimetabolites.

The above discussion provides a factual basis for the use of the methods and probed of the present invention. The methods used with and the utility of the present invention can be shown by the following non-limiting examples and accompanying figures.

### EXAMPLES

### GENERAL METHODS:

**General methods in molecular biology:** Standard molecular biology techniques known in the art and not specifically described were generally followed as in Sambrook et al., *Molecular Cloning: A Laboratory Manual,* Cold Spring Harbor Laboratory Press, New York (1989), and in Ausubel et al., *Current Protocols in Molecular Biology,* John Wiley and Sons, Baltimore, Maryland (1989) and in Perbal, *A Practical Guide to Molecular Cloning,* John Wiley & Sons, New York (1988), and in Watson et al., *Recombinant DNA,* Scientific American Books, New York and in Birren et al (eds) *Genome Analysis: A Laboratory Manual Series, Vols. 1-4* Cold Spring Harbor Laboratory Press, New York (1998) and methodology as set forth in United States patents 4,666,828; 4,683,202; 4,801,531; 5,192,659 and 5,272,057 and incorporated herein by reference. Polymerase chain reaction (PCR) was carried out generally as in *PCR Protocols: A Guide To Methods And Applications,* Academic Press, San Diego, CA (1990). In-situ (In-cell) PCR in combination with Flow Cytometry can be used for detection of cells containing specific DNA and mRNA sequences (Testoni et al, 1996, Blood 87:3822.)

### Recombinant Protein Purification

Marshak et al, "Strategies for Protein Purification and Characterization. A laboratory course manual." CSHL Press, 1996.

### EXAMPLE 1:

### 1. Preparation biotinylated sequence-specific probes.

40ul (100pmol/ul) DNA primers StsC03, StsC04, lPNS05, IPNS06 were labeled with S-S photobiotin (Vector Inc., CA) according to the manufacturer's instructions. These primers were designed for the amplification of the stsC and ipns genes of *S*. *griseus* and *S*. *clavuligerus,* respectively. After n-butanol concentration and EtOH precipitation, the primers were diluted in 200ul water (20pmol/ul). PCR reactions were carried out from pT7 blue3 (Novagene) plasmids containing the previously cloned probe sequences pT7stsC (*stsC* from *S. griseus)* and pT7ipns (*ipns* from *S*. *clavuligerus)* as templates.

Reaction mixtures contained the following: 2ul primer 1 (20pmol/ul) (StsC03 or IPNS05), 2ul primer2 (20pmol/ul) (StSC04 or IPNS06), 2ul template DNA (10-100ng/ul) (pT7blue3/StsC/S.gr or pT7blue/IPNS/S.cl), 2ul buffer, 2ul dNTP mix (2mM), 10ul water, and 0.2ul (0.2u) Taql. Cycling was conducted as follows: five minutes at 95°C, 30 seconds at 98°C, 30 seconds 52°C, one minute 70°C, repeated 34 times. Finally, the reaction was heated to 70°C for ten minutes followed by holding at 4°C until analysis of products was performed.

10 ug of the mixture PCR product was purified on Avidin DLA beads (Vector) to separate biotinylated and non-biotinylated probes. The yield of biotinylated probe was 4-5% (0.4-0.5 ug, Figures 12 and 13). The biotinylated fraction of the probe was used for RecA capturing and non-biotinylated probe was used for alk-direct labeling (Amersham) in hybridization screening.

### 2. RecA capturing of specific target gene containing cosmids from pFD666/S.gr/library.

5ul (0.1ug) of the biotinylated probe was denatured by incubating for ten minutes at 99°C mix with 50ul RecA buffer (25mM TrisAc, pH 7.5, 10mM MgOAc, 2mM CoCl₂, 1 mM ATP, 2mM ATPγS, 5ul (2ug/ul) RecA (NEB). The mixture was then incubated at 37°C for 30 minutes. 2.5ul (2ug/ul) of the CsCl purified cosmid DNA was then added and incubated for an additional hour at 37°C. 5ul (50ng/ul) of lambda Hindlll digested DNA was added to the mixture to remove excess RecA, incubated for ten minutes at 37°C followed by the addition of 2ug/ul Proteinase K and SDS 0.2%. Enzymatic digestion was carried out for 30 minutes at 37°C and then reaction was stopped by adding PMSF (100mM) to a final concentration of 3mM.

The captured DNA was separated on Avidin DLA beads (20ul) beads prepared according to the manufacturer's instructions. At the final step captured DNA was eluted with 100-200 ul (0.1 M NaOH, 1mM EDTA), EtOH precipitated, and dissolved in 20ul water. DNA was electrotransformed into E. *coli* XL1 (Stratagene). Positive clones were detected by colony hybridization with alk-direct StsC probes (Figure 14). DNA from positive clones were purified and verified by dot-blot, southern hybridization, PCR and bacterial growth on LB agar with streptomycin (20ug/ul) plates.

### 3. Direct cloning of RecA captured DNA fragments from S. griseus chromosomal DNA.

RecA capture was carried out as described above, but instead of cosmid DNA, 5ul (1ug/ul) of chromosomal DNA from *S*. *griseus* digested with Mbol/Sau3AI and CIP was used. After RecA capturing and binding to the Avidin DLA beads, DNA was eluted with 200ul 2.5mM biotin and directly ligated into the pSCOS1 cosmid vector (Strategene). After packaging into lambda extracts clones were plated on LB agar with Amp (50ug/ml) and Km (25ug/ml). Positive clones were detected by colony hybridization with alk-direct StsC probes (Figure 14). DNA from positive clones was purified and verified by dot-blot, southern hybridization, PCR and bacterial growth on LB agar with streptomycin (20µg/µl) plates. Positive clones most often contain related pathway genes, as confirmed by additional hybridization with related gene probes, such as *strD, strB,* and *stsC* (Figure 15), and PCR .(Figure 16). Additionally, heterologous expression of these genes is often observed, as judged by antibiotic resistance (Figure 17) and HPLC chromatographic profiling of cell extracts and fermentation broths, further demonstrating the utility of this invention for expression cloning screening.

Results from both examples clearly demonstrate the advantages of targeted cloning in providing highly enriched libraries of specific genes and associated sequences, including biosynthetic pathways. Library enrichment of several hundred fold for specific genes and related biosynthetic pathways it has been demonstrated by use of the present invention (Figure 14, 18).

Throughout this application, various publications, including United States patents, are referenced by author and year and patents by number. Full citations for the publications are listed below, in order to more fully describe the state of the art to which this invention pertains.

The invention has been described in an illustrative manner, and it is to be understood that the terminology which has been used is intended to be in the nature of words of description rather than of limitation.

Obviously, many modifications and variations of the present invention are possible in light of the above teachings. It is, therefore, to be understood that within the scope of the described invention, the invention can be practiced otherwise than as specifically described but within the scope of the claims.

**TABLE I**

| **List of example degenerate PCR primers of target gene cloning** | | | | |
|---|---|---|---|---|
| **Gene** | **Function** | **Pathway** | **Name** | **Sequence (5' to 3')** |
| aac6' | Aminoglycoside 6'-N-acetyltransferase | aminoglycoside | AAC6001 | CGATGCTSTAYGARTGGCTA |
| | | | AAC6002 | TGGCGYGTYTGVACCATGTA |
| | | | AAC6003 | CCGACRCTYGCKGACGTACA |
| | | | AAC6004 | CATCBGGSGTSGTTACGGTA |
| | | | | |
| acvs | alpha-aminoadipyl-L-cysteinyl-D-valine synthetase | beta-lactam | ACVS3 | TGCTSGTSGGSGARGAGCTGA |
| | | | ACVS4 | TCSACYTTGCCRTTGACRTTGA |
| | | | ACVS5 | TTCACCGARRCSGCGTTCGTCA |
| | | | ACVS6 | CGCCGGSACCATSAYCCGGATSA |
| | | | | |
| *dhfr2* | trimethoprim resistance | | DHFR2-1 | GATCGCGTGCGCAAGAAAT |
| | | | DHFR2-2 | CCACSTTTGGYHTRGGRGATCG |
| | | | DHFR2-3 | AYRCGTTCAAGYGCMGCMACAG |
| | | | DHFR2-4 | GATAAATYTGYACTGARCCK |
| | | | | |
| *ipns* | isopenicillin N synthase | beta-lactam | IPNS3 | TTCKSCGAGGACCACCCGMWGAT |
| | | | IPNS4 | GGAAGTAGTCGTKSGTGAKGT |
| | | | IPNS5 | GATGCACGAGGTBAACVTCT |
| | | | IPNS6 | TCGGWASAGSACGGTGATCA |
| | | | | |
| NIM | Nitroimidazole resistance | | N101 | ATGCSRCGYAARCGGCARTTGT |
| | | | N102 | CGATRGCYTCYTGCCYGTCAT |
| | | | N103 | CGTTGGCYCTTCATGGSGATGA |
| | | | N104 | CCTTTGGCTATYTCRGCYTCCAT |
| | | | | |
| PKS | Polyketide synthase | | PKS1 | GAGTTCGACGCSGVSTTCTT |
| | | | PKS2 | GGTGTGNCCGATGTTGGACTT |
| | | | PKS3 | GCARCGVCTCCTGCTSGAAA |
| | | | PKS4 | TTGCTSGCRCCGTCCTGGTT |
| | | | | |
| *strB1* | Amidinotrasferase I | aminoglycoside | StrB1001 | AGCGTSTTCGCSGTGGAGTA |
| | | | StrB1002 | GTGCTTCTCSAGMAGCTTGA |
| | | | StrB1003 | TSAAGGAGACCGARGASGAA |
| | | | StrB 1004 | CGSACCACSAGCAGGTTCA |
| | | | | |
| *strD* | dTDP-glucose synthase | aminoglycoside | StrD1 | CTTCTAYGSVCTGGAGGCCA |
| | | | StrD2 | GAGGRGATCTGSSCCDTGCT |
| | | | StrD3 | GTGGGMGACGGYTCSAARTT |
| | | | StrD4 | TYGCGCAGCACGATGGAGAA |
| | | | | |
| *strE* | dTDP-glucose dehydratase | aminoglycoside | StrE1 | CACTACGTCCRSACCCTCCT |
| | | | StrE2 | GCCAKBCCGTCGSYCAGGT |
| | | | StrE3 | CSGYSTTCGTGCGGACCAA |
| | | | StrE4 | CGGTCSKSGACGTRCTCCA |
| | | | | |
| *stsA* | L-alanine:N-amidino-3-keto-scyllo- | aminoglycoside | StsA1 | ATCGTSCCYGGRTASATGTT |
| | inosamine aminotransferase | | StsA2 | GATGGARCGSCCSAGSACA |
| | | | StsA3 | CTTSTCCCTSAAYCASTAYAAGA |
| | | | StsA4 | GCGTTCCRGGYTCRAAGGAA |
| | | | | |
| *stsC* | pyridoxal phosphate dependent | aminoglycoside | StsC1 | GGMTSCCSSTSATCGAGGACT |
| | amidotransferase | | StsC2 | GTCGAACCGGGSMSGGGTCCA |
| | | | StsC3 | CGGCGCRYSGGRGTSTTCA |
| | | | StsC4 | GTWCAGCCGGGTGKCGTTCA |
| | | | | |
| Taxol | taxadiene synthase | | Taxo101 | ATGATGGGTYTGSTCSAGA |
| | | | Taxo102 | TTTYTCRTCSCCYGTRTTCAT |
| | | | Taxo103 | TCCYGGYCCKGTSGTMATGAT |
| | | | Taxo104 | ACCYTCSAASGCRTTYAACAT |

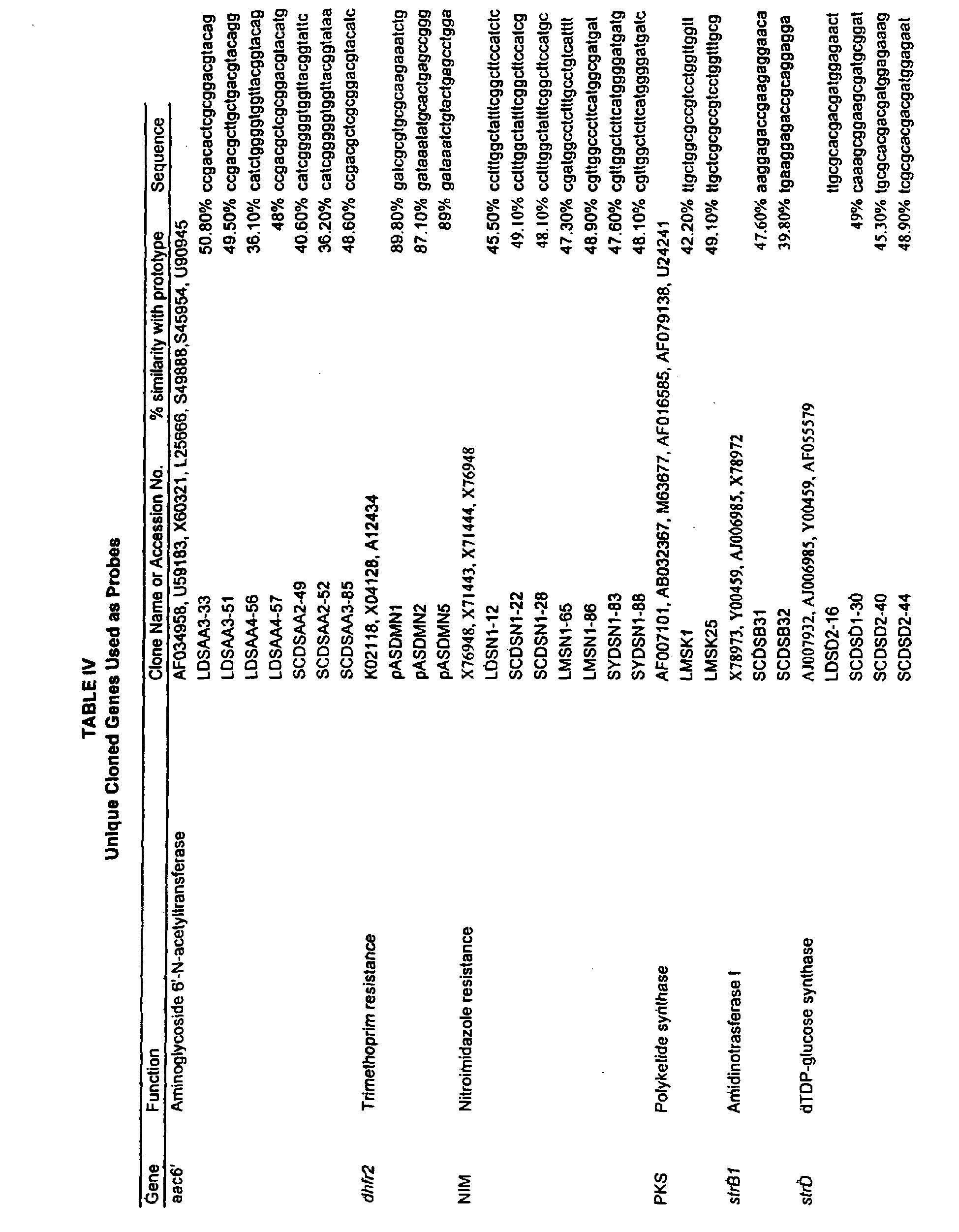

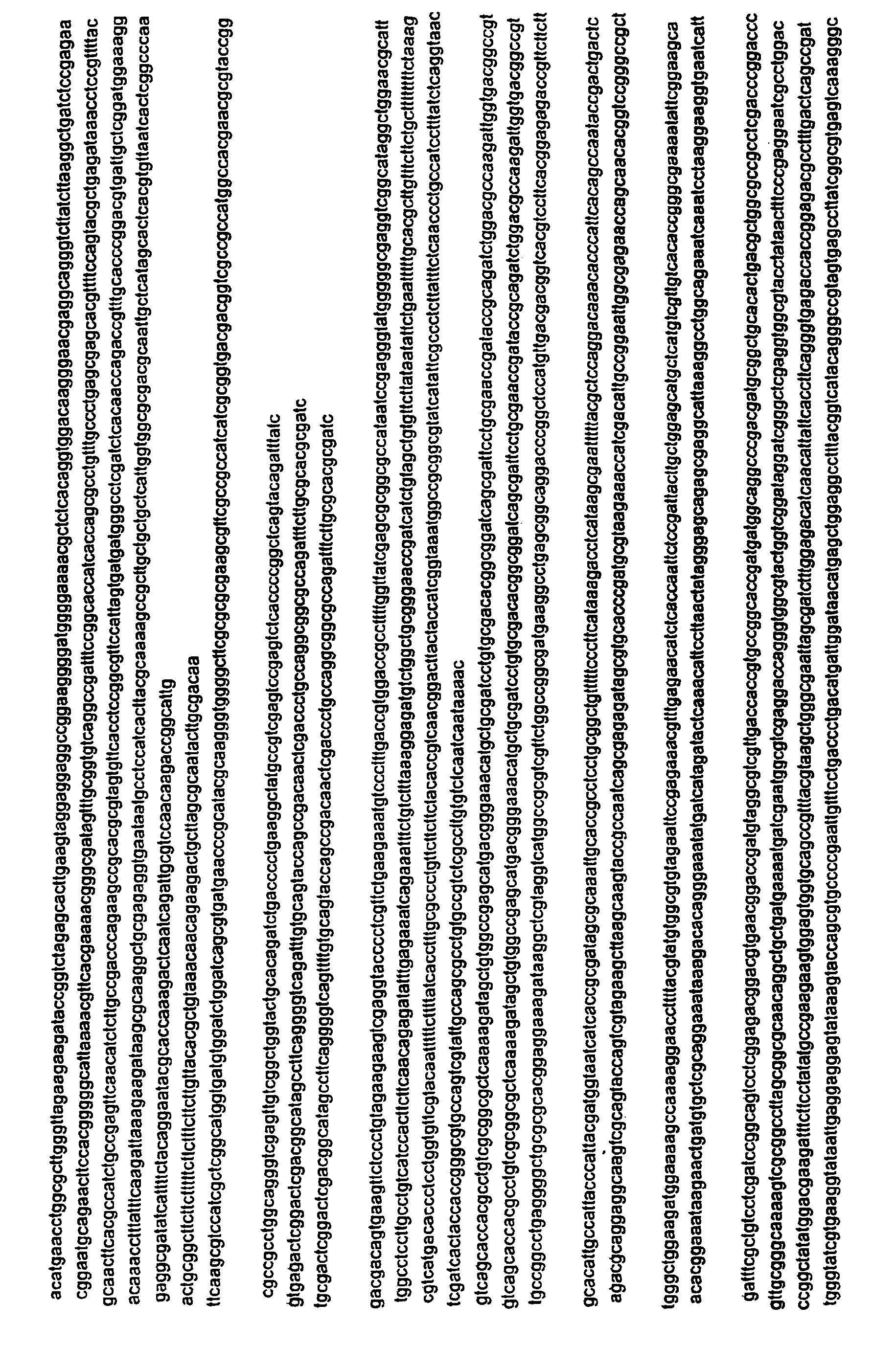

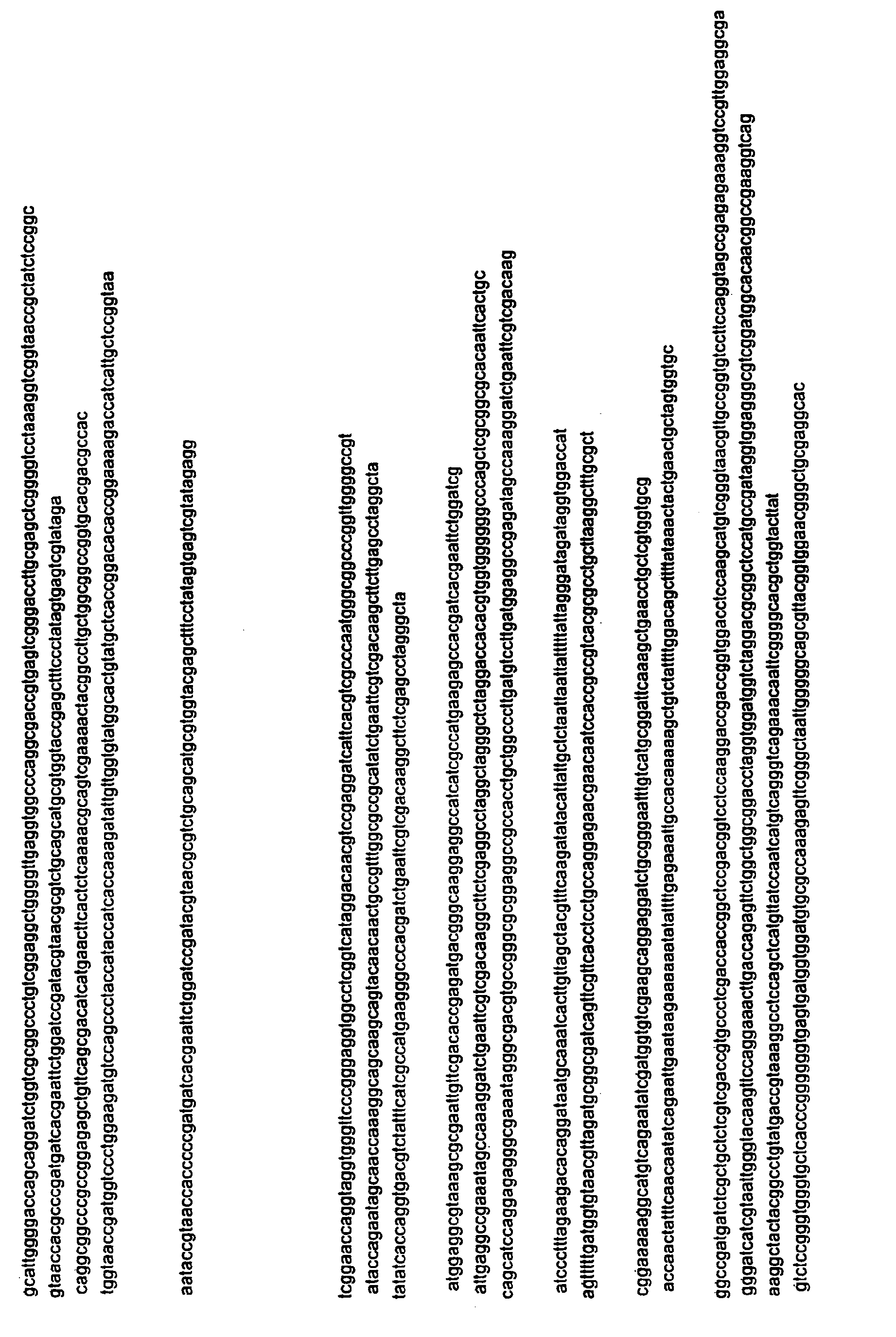

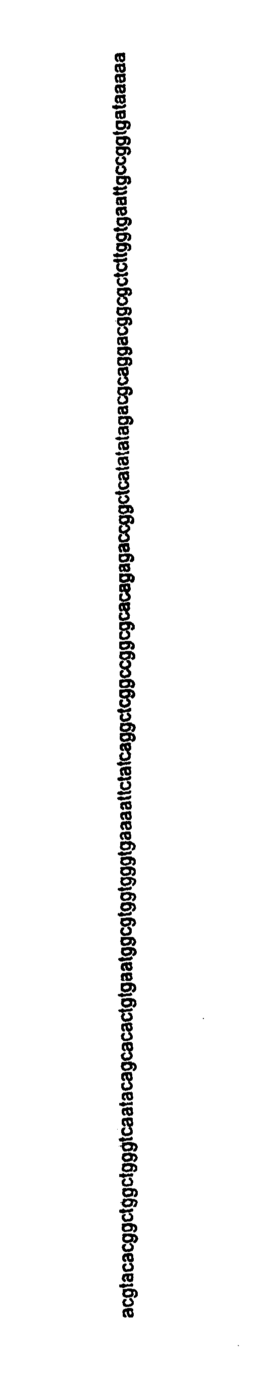

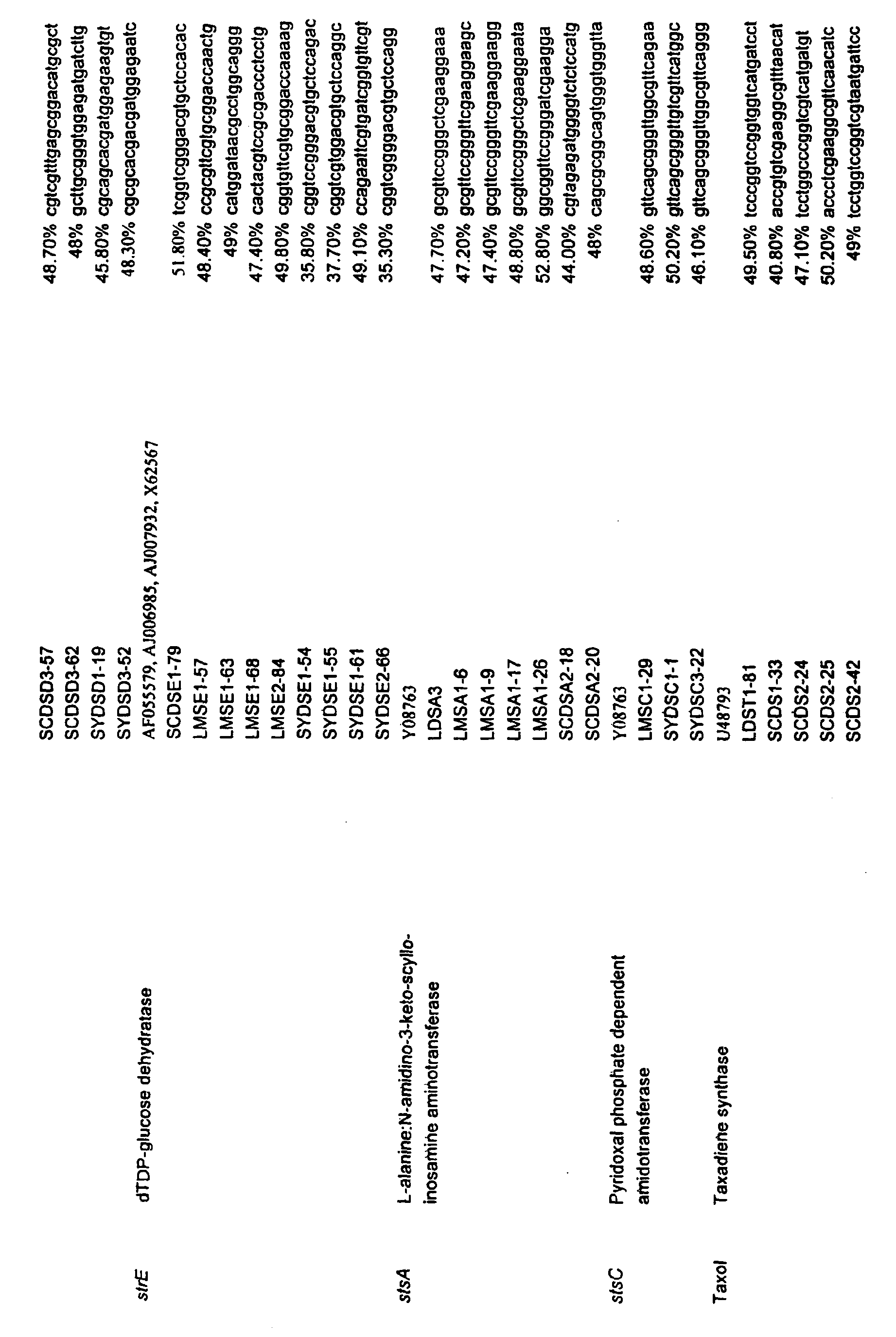

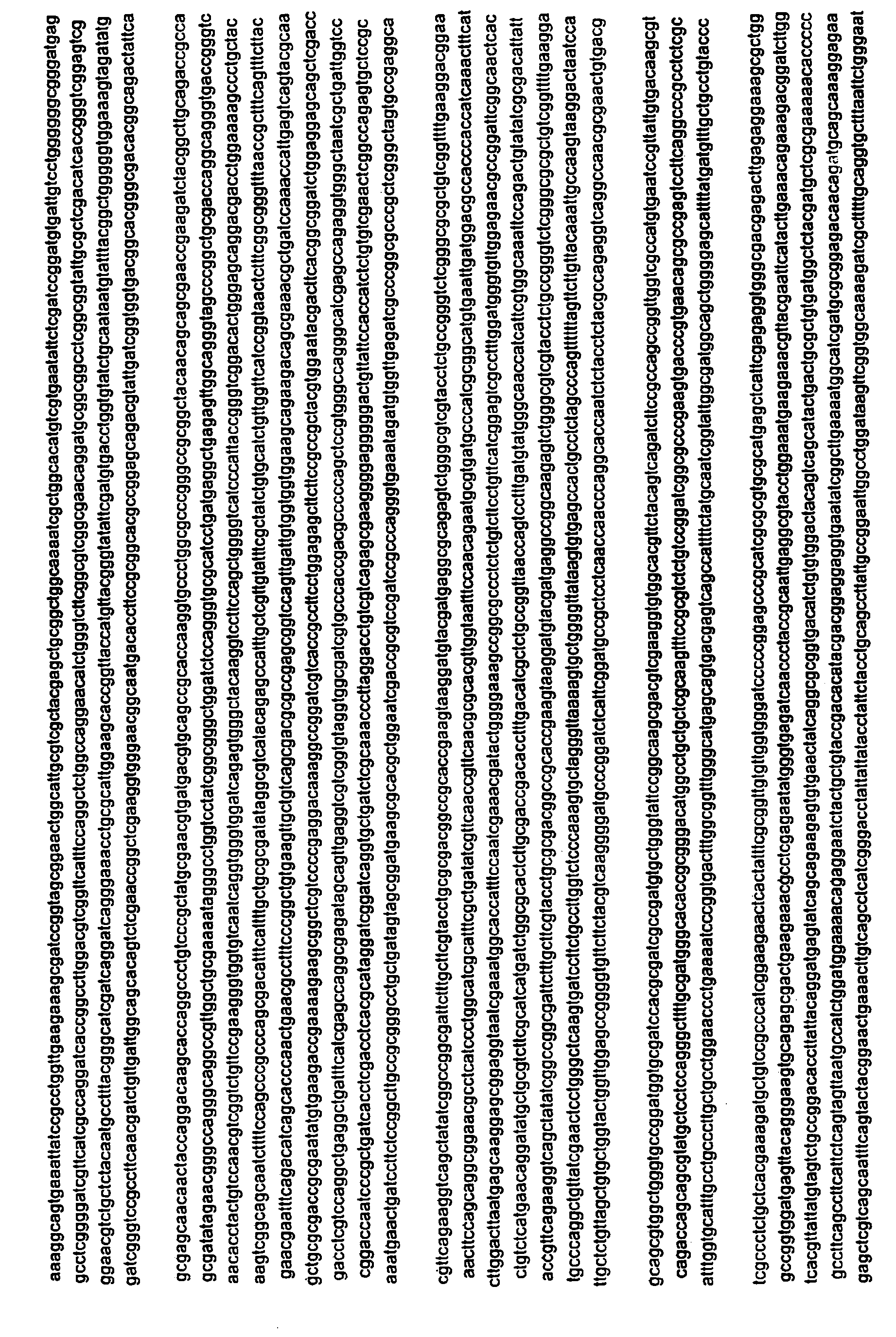

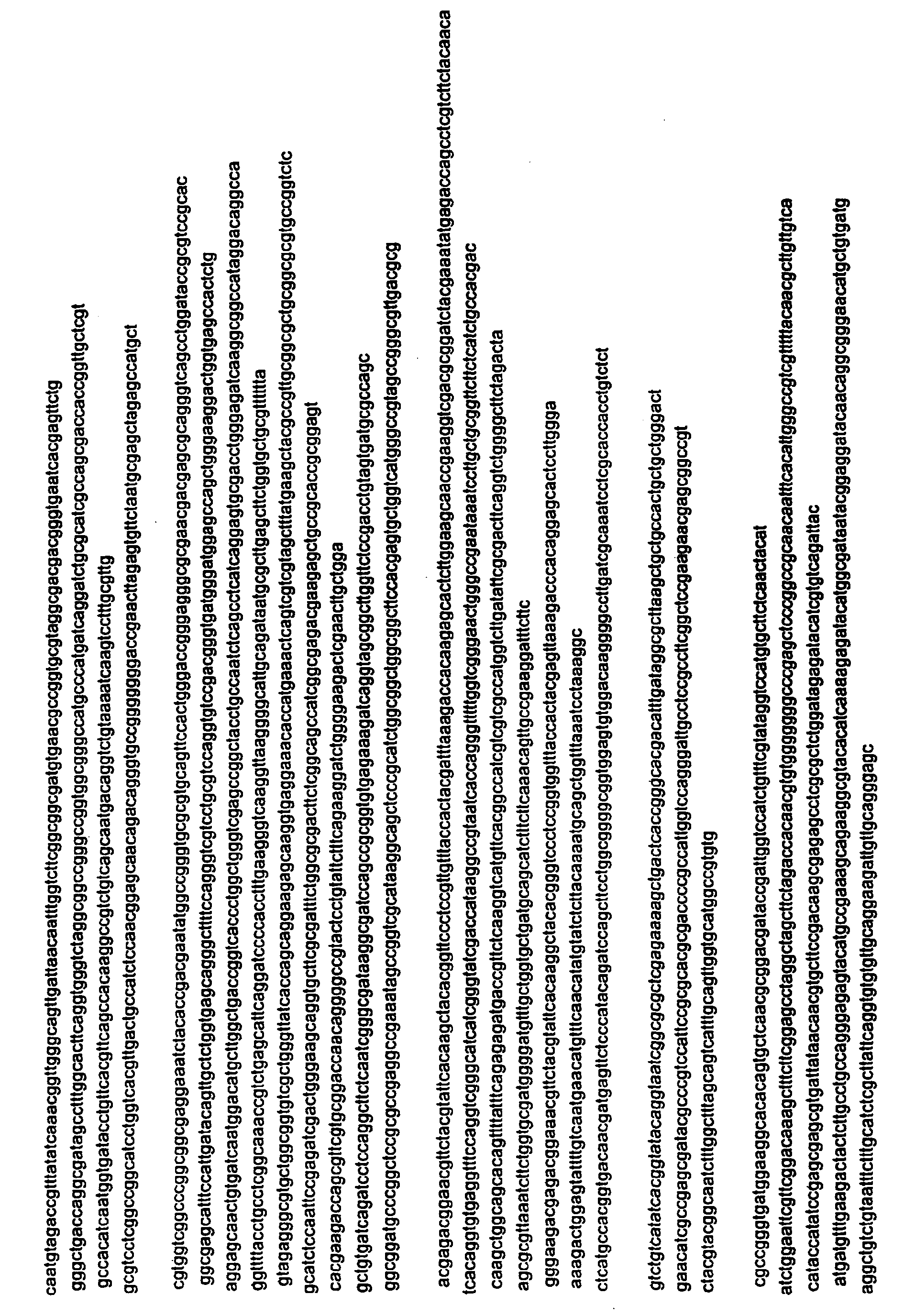

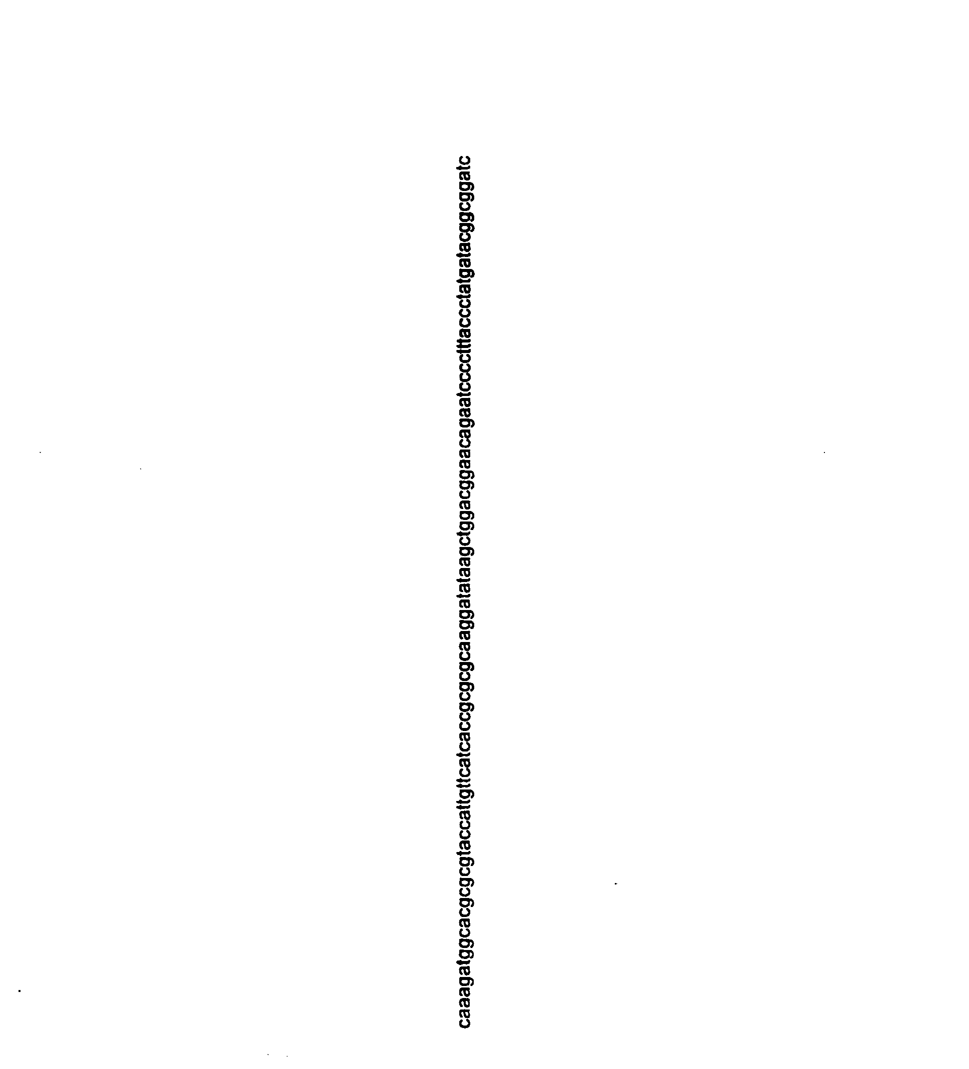

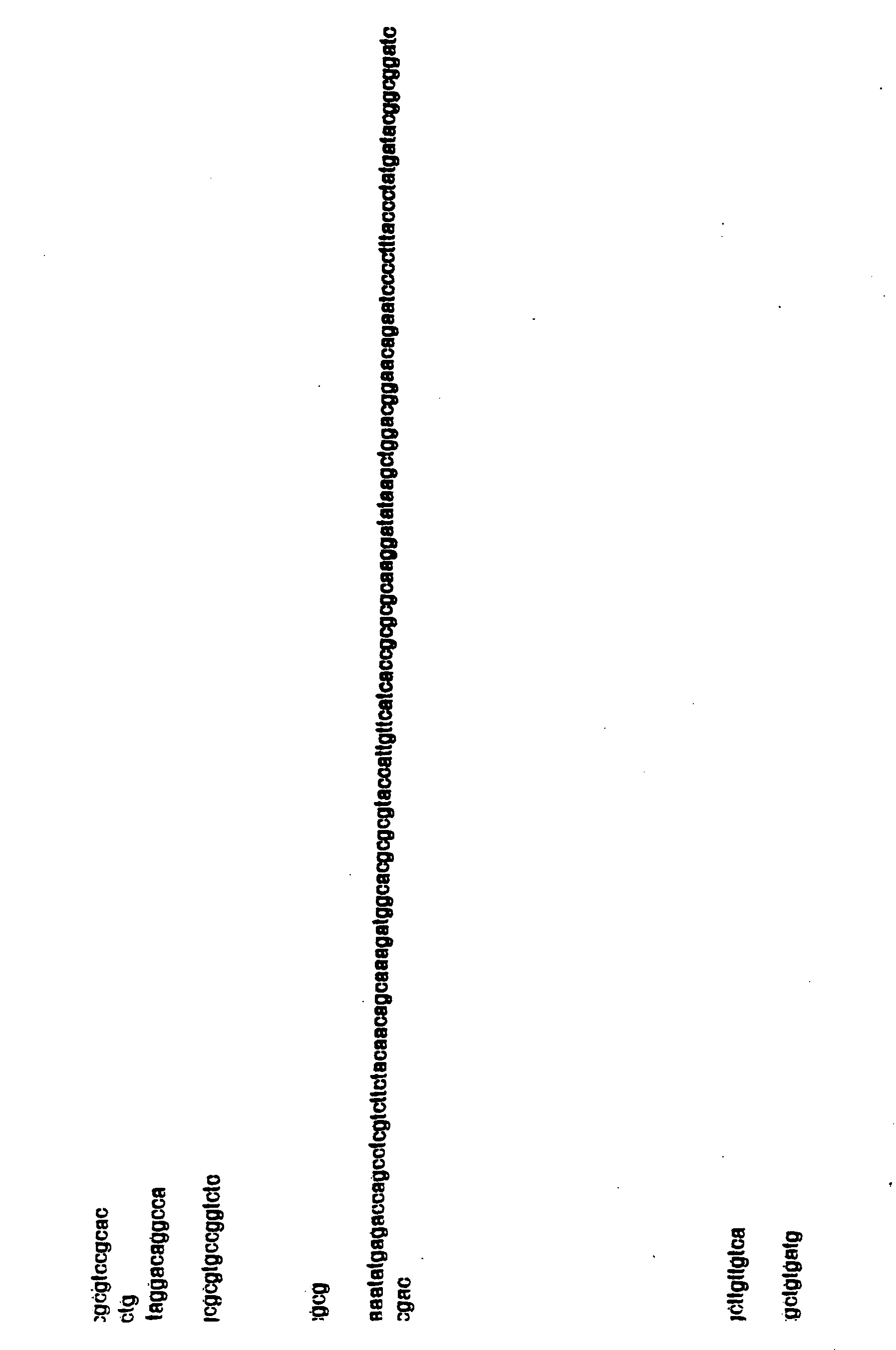

### REFERENCES

Burke and Olson, "Preparation of Clone Libraries in Yeast Artificial-Chromosome Vectors" in Methods in Enzymology, Vol. 194, "Guide to Yeast Genetics and Molecular Biology", eds. C. Guthrie and G. Fink, Academic Press, Inc., Chap. 17, pp. 251-270 (1991).
Capecchi, "Altering the genome by homologous recombination" Science 244:1288-1292 (1989).
Davies et al., "Targeted alterations in yeast artificial chromosomes for inter-species gene transfer", Nucleic Acids Research, Vol. 20, No. 11, pp. 2693-2698 (1992).
Dickinson et al., "High frequency gene targeting using insertional vectors", Human Molecular Genetics, Vol. 2, No. 8, pp. 1299-1302 (1993).
Duff and Lincoln, "Insertion of a pathogenic mutation into a yeast artificial chromosome containing the human APP gene and expression in ES cells", Research Advances in Alzheimer's Disease and Related Disorders, 1995.
Huxley et al., "The human HPRT gene on a yeast artificial chromosome is functional when transferred to mouse cells by cell fusion", Genomics, 9:742-750 (1991).
Jakobovits et al., "Germ-line transmission and expression of a human-derived yeast artificial chromosome", Nature, Vol. 362, pp. 255-261 (1993).
Lamb et al., "Introduction and expression of the 400 kilobase *precursor amyloid protein* gene in transgenic mice", Nature Genetics, Vol. 5, pp. 22-29 (1993).
Pearson and Choi, *Expression of the human b-amyloid precursor protein gene from a yeast artificial chromosome in transgenic mice.* Proc. Natl. Acad. Sci. USA, 1993. **90**:10578-82.
Rothstein, "Targeting, disruption, replacement, and allele rescue: integrative DNA transformation in yeast" in Methods in Enzymology, Vol. 194, "Guide to Yeast Genetics and Molecular Biology", eds. C. Guthrie and G. Fink, Academic Press, Inc., Chap. 19, pp. 281-301 (1991).
Schedl et al., "A yeast artificial chromosome covering the tyrosinase gene confers copy number-dependent expression in transgenic mice", Nature, Vol. 362, pp. 258-261 (1993).
Strauss et al., "Germ line transmission of a yeast artificial chromosome spanning the murine a, (I) collagen locus", Science, Vol. 259, pp. 1904-1907 (1993).
Gilboa, E, Eglitis, MA, Kantoff, PW, Anderson, WF: Transfer and expression of cloned genes using retroviral vectors. BioTechniques 4(6):504-512, 1986.
Cregg JM**,** Vedvick TS, Raschke WC: Recent Advances in the Expression of Foreign Genes in *Pichia pastoris,* Bio/Technology 11:905-910, 1993
Culver, 1998. Site-Directed recombination for repair of mutations in the human ADA gene. (Abstract) Antisense DNA & RNA based therapeutics, February, 1998, Coronado, CA.
Huston et al, 1991 "Protein engineering of single-chain Fv analogs and fusion proteins" in Methods in Enzymology (JJ Langone, ed.; Academic Press, New York, NY) 203:46-88.
Johnson and Bird, 1991 "Construction of single-chain Fvb derivatives of monoclonal antibodies and their production in *Escherichia coli* in Methods in Enzymology (JJ Langone, ed.; Academic Press, New York, NY) 203:88-99.
Mernaugh and Mernaugh, 1995 "An overview of phage-displayed recombinant antibodies" in Molecular Methods In Plant Pathology (RP Singh and US Singh, eds.; CRC Press Inc., Boca Raton, FL) pp. 359-365.

## Claims

1. A method of cloning an entire family of genes from a mixed DNA sample by:
creating at least one primer for a targeted gene sequence;
adding the primer to a sample of DNA;
performing degenerate, nested PCR to replicate the targeted gene sequence and to create a gene template;
adding the gene template to the sample of DNA to facilitate stable hybridization with similar sequences; and
isolating an entire family of genes or gene containing gene clusters by purification selected from the group consisting of gel purification and affinity purification.

2. The method according to claim 1, wherein said creating step further includes creating a primer using k-tuple, template derivation, and degenerate PCR.

3. The method according to claim 1, wherein said performing step further includes the step of labelling the replicated gene template with an affinity tag.

4. A method of isolating coding genes or gene clusters for at least one polypeptide that produces trimethoprim by directly isolating and subsequent cloning at least one coding gene or gene clusters coding for at least one polypeptide that produces trimethoprim, wherein said isolating step includes the steps of:
creating at least one primer for a targeted gene sequence using a method selected from the group consisting of k-tuple, template derivation, and degenerate PCR;
adding the primer to a sample of DNA;
performing degenerate, nested PCR to replicate the targeted gene sequence and to create a gene template;
adding the gene template to the sample of DNA to facilitate stable hybridization with similar sequences; and
isolating an entire family of genes or gene containing gene clusters coding for trimethoprim by purification selected from the group consisting of gel purification and affinity purification.

5. The method according to claim 4, wherein said creating step further includes creating a primer containing a target oligonucleotides coding for at least a portion of DHFR2.

6. A method of directly isolating targeted genes or gene clusters by:
creating at least one primer for a targeted gene sequence;
adding the primer to a sample of DNA;
performing degenerate, nested PCR to replicate the targeted gene sequence and to create a gene template:
adding the gene template to the sample of DNA to facilitate stable hybridization with similar sequences; and
isolating an entire family of gene containing gene clusters by purification selected from the group consisting of gel purification and affinity purification.

## Patentansprüche

1. Verfahren zum Klonieren einer ganzen Familie von Genen aus einer gemischten DNA Probe, durch:
Erzeugen von mindestens einem Primer für eine als Ziel ausgewählte Gensequenz;
Zugeben des Primers zu einer DNA Probe;
Durchführen einer degenerierten, verschachtelten PCR zur Replikation der als Ziel ausgewählten Gensequenz und zur Erzeugung einer Genmatrize;
Zugeben der Genmatrize zur DNA Probe zur Erleichterung einer stabilen Hybridisierung mit ähnlichen Sequenzen; und
Isolieren einer ganzen Familie von Genen oder eines Gens mit Genclustern durch eine Reinigung, die aus der Gruppe bestehend aus Gelreinigung und Affinitätsreinigung ausgewählt ist.

2. Verfahren nach Anspruch 1, wobei der Erzeugungsschritt weiter das Erzeugen eines Primers mittels k-Tuple, Matrizenableitung und degenerierter PCR aufweist.

3. Verfahren nach Anspruch 1, wobei der Durchführungsschritt weiter den Schritt des Markierens der replizierten Genmatrize mit einem Affinitäts-Tag aufweist.

4. Verfahren zum Isolieren von kodierenden Genen oder Gen-Clustern für mindestens ein Polypeptid, das Trimethoprim erzeugt, durch direktes Isolieren und anschließendes Klonieren von mindestens einem kodierenden Gen oder Gen-Clustern, das bzw. die mindestens ein Trimethoprim erzeugendes Polypeptid kodieren, wobei der Isolierungsschritt die folgenden Schritte aufweist:
Erzeugen von mindestens einem Primer für eine als Ziel ausgewählte Gensequenz mittels eines Verfahrens, das aus der Gruppe bestehend aus k-Tuple, Matrizenableitung und degenerativer PCR ausgewählt ist;
Zugeben des Primers zu einer DNA Probe;
Durchführen einer degenerierten, verschachtelten PCR zur Replikation der als Ziel ausgewählten Gensequenz und zur Erzeugung einer Genmatrize;
Zugeben der Genmatrize zur DNA Probe zur Erleichterung einer stabilen Hybridisierung mit ähnlichen Sequenzen; und
Isolieren einer ganzen Familie von Genen oder eines Gens mit Genclustern, die für Trimethoprim kodieren, durch eine Reinigung, die aus der Gruppe bestehend aus Gelreinigung und Affinitätsreinigung ausgewählt ist.

5. Verfahren nach Anspruch 4, wobei der Erzeugungsschritt weiter das Erzeugen eines Primers umfasst, der Zieloligonukleotide enthält, die für mindestens einen Abschnitt von DHFR2 kodieren.

6. Verfahren zum direkten Isolieren von als Ziel ausgewählten Genen oder Gen-Clustern durch:
Erzeugen von mindestens einem Primer für eine als Ziel ausgewählte Gensequenz;
Zugeben des Primers zu einer DNA Probe;
Durchführen einer degenerierten, verschachtelten PCR zur Replikation der als Ziel ausgewählten Gensequenz und zur Erzeugung einer Genmatrize;
Zugeben der Genmatrize zur DNA Probe zur Erleichterung einer stabilen Hybridisierung mit ähnlichen Sequenzen; und
Isolieren einer ganzen Familie eines Gens mit Genclustern durch eine Reinigung, die aus der Gruppe bestehend aus Gelreinigung und Affinitätsreinigung ausgewählt ist.

## Revendications

1. Procédé de clonage d'une famille entière de gènes à partir d'un échantillon d'ADN mixte, par :
la création d'au moins une amorce pour une séquence de gène ciblé ;
l'ajout de l'amorce à un échantillon d'ADN;
la réalisation d'une PCR dégénérée nichée afin de répliquer la séquence du gène ciblé et de créer une matrice de gène ;
l'ajout de la matrice de gène à l'échantillon d'ADN afin de faciliter une hybridation stable avec des séquences similaires ; et
l'isolement d'une famille entière de gènes ou de clusters de gènes contenant des gènes par une purification choisie dans le groupe constitué par la purification sur gel et la purification par affinité.

2. Procédé selon la revendication 1, **caractérisé en ce que** ladite étape de création inclut en outre la création d'une amorce en utilisant des k-tuples, une dérivation de matrice et une PCR dégénérée.

3. Procédé selon la revendication 1, **caractérisé en ce que** ladite étape de réalisation inclut en outre l'étape de marquage de la matrice de gène répliquée avec une étiquette d'affinité.

4. Procédé d'isolement de gènes ou de clusters de gènes codant pour au moins un polypeptide qui produit du triméthoprime, par isolement direct et clonage subséquent d'au moins un gène codant ou de clusters de gènes codant pour au moins un polypeptide qui produit du triméthoprime, **caractérisé en ce que** ladite étape d'isolement inclut les étapes de :
création d'au moins une amorce pour une séquence de gène ciblé en utilisant un procédé choisi dans le groupe constitué par des k-tuples, une dérivation de matrice et une PCR dégénérée ;
ajout de l'amorce à un échantillon d'ADN ;
réalisation d'une PCR dégénérée nichée afin de répliquer la séquence du gène ciblé et de créer une matrice de gène ;
ajout de la matrice de gène à l'échantillon d'ADN afin de faciliter une hybridation stable avec des séquences similaires ; et
isolement d'une famille entière de gènes ou de clusters de gènes contenant des gènes codant pour le triméthoprime par une purification choisie dans le groupe constitué par la purification sur gel et la purification par affinité.

5. Procédé selon la revendication 4, **caractérisé en ce que** ladite étape de création inclut en outre la création d'une amorce contenant un oligonucléotide cible codant pour au moins une partie de DHFR2.

6. Procédé d'isolement direct de gènes ou de clusters de gènes ciblés par :
la création d'au moins une amorce pour une séquence de gène ciblé ;
l'ajout de l'amorce à un échantillon d'ADN ;
la réalisation d'une PCR dégénérée nichée afin de répliquer la séquence du gène ciblé et de créer une matrice de gène ;
l'ajout de la matrice de gène à l'échantillon d'ADN afin de faciliter une hybridation stable avec des séquences similaires ; et
l'isolement d'une famille entière de clusters de gènes contenant des gènes par une purification choisie dans le groupe constitué par la purification sur gel et la purification par affinité.
